(19) Europäisches Patentamt
European Patent Office
Office européen des brevets

(11) Numéro de publication: **0 421 890 B1**

# (12) FASCICULE DE BREVET EUROPEEN

(45) Date de publication de fascicule du brevet: **25.01.95**

(51) Int. Cl.[6]: **C07D 211/90**, C08K 5/3435

(21) Numéro de dépôt: **90420291.8**

(22) Date de dépôt: **19.06.90**

(54) **Nouvelles dihydropyridines à groupements amine encombrée.**

(30) Priorité: **02.10.89 FR 8913053**

(43) Date de publication de la demande:
**10.04.91 Bulletin 91/15**

(45) Mention de la délivrance du brevet:
**25.01.95 Bulletin 95/04**

(84) Etats contractants désignés:
**AT BE CH DE DK ES FR GB GR IT LI LU NL SE**

(56) Documents cités:
**EP-A- 0 002 260**
**US-A- 4 214 088**

(73) Titulaire: **GREAT LAKES CHEMICAL FRANCE S.A.**
**5, rue de la Grand Ourse**
**Cergy Saint-Christophe**
**F-95800 Cergy Pontoise (FR)**

(72) Inventeur: **Carette, Louis**
**64 rue de l'Egalité,**
**Résidence Rodin - Appt.1411**
**F-92130 Issy les Moulineaux (FR)**
Inventeur: **Gay, Michel**
**10, rue Henri Rolland**
**F-69100 Villeurbanne (FR)**
Inventeur: **Lavault, Sylvie**
**17, rue Jassron**
**F-69003 Lyon (FR)**
Inventeur: **Mur, Gilles**
**176, rue de Paris**
**F-94190 Villeneuve,**
**St, Georges (FR)**

(74) Mandataire: **Koch, Gustave et al**
**Cabinet PLASSERAUD**
**84, rue d'Amsterdam**
**F-75009 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 421 890 B1

Rank Xerox (UK) Business Services
(3.10/3.09/3.3.3)

## Description

La présente invention concerne de nouveaux composés dérivant de la dihydropyridine et comportant des groupements pipéridinyle substitué.

Elle concerne également l'utilisation de ces composés pour la stabilisation thermique et lumière des polymères.

Le brevet FR-A-2 239 496 décrit des diméthyl-2,6 dicarboxylate-3,5 dihydro-1,4 pyridines comme stabilisants thermiques du polychlorure de vinyle (PVC).

Le brevet EP-A-0 005 678 décrit une synergie entre les diméthyl-2,6 dicarboxylate-3,5 dihydro-1,4 pyridines et les $\beta$-dicétones pour la stabilisation thermique du PVC

Ces composés de la dihydro-1,4 pyridine ont une bonne efficacité pour la stabilisation thermique du PVC. Ils sont cependant insuffisants pour certaines applications où le polymère est soumis à des expositions extérieures, c'est-à-dire lorsqu'il est nécessaire d'avoir une stabilité lumière.

Le brevet EP-A-0 002 260 décrit des dérivés de l'acide malonique de pipéridines encombrés stériquement comme stabilisants contre la dégradation induite par la lumière.

La présente invention concerne de nouveaux composés à fonction dihydro-1,4 pyridine de formule générale (Ia) ou (Ib) :

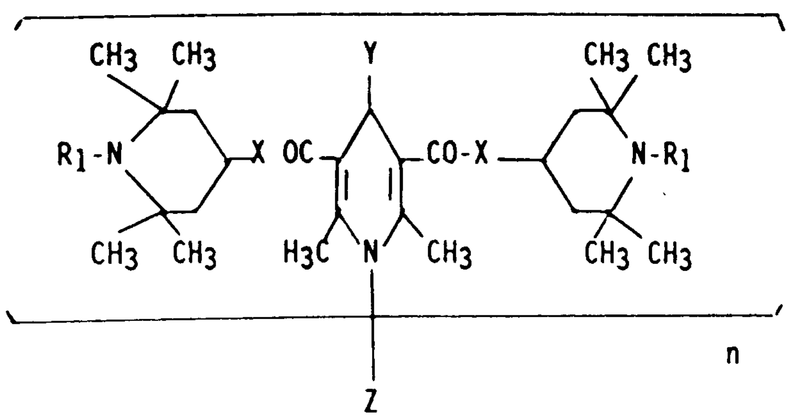

(Ia)

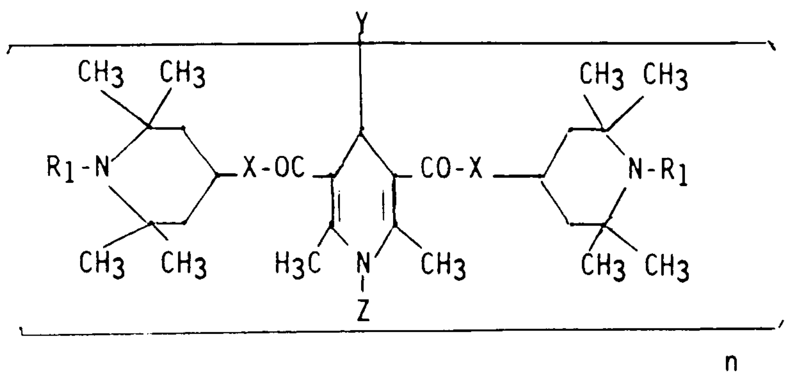

(Ib)

dans lesquelles :

- n est 1 ou 2
- $R_1$ représente un atome d'hydrogène, un radical méthyle ou un radical acétyle ;
- X représente un atome d'oxygène ou un radical N-$R_2$ dans lequel $R_2$ représente un atome d'hydrogène ou un radical alkyle ayant 1 à 18 atomes de carbone ;
- Y représente un atome d'hydrogène, un radical alkyle ayant 1 à 18 atomes de carbone ou un radical phényle ;
- Z représente un atome d'hydrogène ou un radical alkyle ayant 1 à 18 atomes de carbone ;
- lorsque n = 2 l'un des 2 symboles Y et Z peut représenter un radical alkylène ayant 1 à 12 atomes de carbone.

Les composés de formule (Ia) et (Ib) peuvent être préparés notamment selon la méthode de HANTZSCH [CHEMICAL REVIEWS 72, 1 (1972)] en faisant réagir un ester acétoacétique ou un acétoacétamide de formule (II)

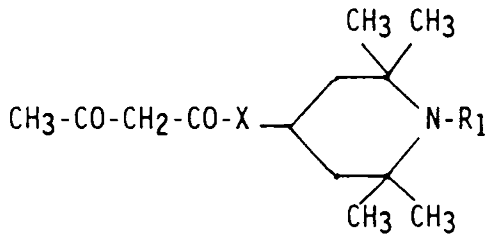

(II)

avec un aldéhyde de formule (III) Y{CHO$)_n$ et une amine de formule (IV) Z{$NH_2)_n$,
les différents symboles n, X, $R_1$, Y et Z dans les formules (II), (III) et (IV) ayant les significations indiquées précédemment pour les formules (Ia) et (Ib).

On peut utiliser par exemple le mode opératoire décrit dans ORGANIC SYNTHESIS, volume XIV, page 30 (1934).

Lorsque X et Z représentent simultanément chacun un atome d'hydrogène, il est souvent plus simple de mettre en oeuvre l'hexaméthylène tétramine plutôt que le mélange formaldéhyde/ammoniac.

Un mode opératoire pour cette variante est décrit dans le brevet SU 300 465.

Les esters acétoacétiques et les acétamides de formule (II) peuvent eux-mêmes être obtenus par réaction du dicétène sur l'hydroxy-4 pipéridine ou l'amino-4 pipéridine correspondante de formule (V)

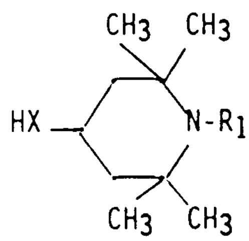

(V)

dans laquelle X représente un atome d'oxygène ou un radical -N-$R_2$, $R_1$ et $R_2$ ayant les significations indiquées précédemment pour les composés de formule (Ia) et (Ib).

Parmi les composés de formule (Ia) ou (Ib) on peut citer comme exemples:

- la diméthyl-2,6 bis[(pentaméthyl-1,2,2,6,6 pipéridinyl-4)-oxycarbonyl]-3,5 dihydro-1,4 pyridine ;
- la diméthyl-2,6 bis[(tétraméthyl-2,2,6,6 pipéridinyl-4)-oxycarbonyl]-3,5 dihydro-1,4 pyridine ;
- la diméthyl-2,6 bis[(tétraméthyl-2,2,6,6 pipéridinyl-4)-amino carbonyl]-3,5 dihydro-1,4 pyridine ;
- la diméthyl-2,6 bis[(tétraméthyl-2,2,6,6 pipéridinyl-4)-N-butylaminocarbonyl]-3,5 dihydro-1,4 pyridine ;
- le bis[diméthyl-2,6 bis[(pentaméthyl-1,2,2,6,6 pipéridinyl-4)-oxycarbonyl]-3,5 dihydro-1,4 pyridinyl-4]-1,6 hexane.

Les composés de formules (Ia) et (Ib) peuvent être utilisés comme stabilisants lumière et stabilisants thermiques dans les polymères organiques;

Ainsi ils peuvent être utilisés comme anti-UV dans les polyoléfines, les polystyrènes, les polyalcadiènes, les polyuréthannes, les polyamides, les polyesters, les polycarbonates, les polysulfones, les polyéthers-sulfones, les polyéthers-cétones, les polymères acryliques, les polymères halogénés, leurs copolymères et leurs mélanges.

Les composés de formule (I) sont plus particulièrement utilisés dans les polyoléfines et les polyalcadiènes tels que le polypropylène, le polyéthylène haute densité, le polyéthylène basse densité, le polyéthylène basse densité linéaire, le polybutadiène, leurs copolymères ou leurs mélanges.

Un autre objet de la présente invention consiste donc dans des compositions de polymère organique stabilisé contre les effets néfastes de la lumière et des rayons ultra-violets par une quantité efficace d'au moins un composé de formule (Ia) ou (Ib).

Habituellement ces compositions contiennent de 0,004 à 20 milliéquivalents de fonction tétraméthyl-2,2,6,6 pipéridinyle pour 100 g de polymère.

De préférence les compositions polymériques stabilisées selon l'invention contiennent de 0,020 à 4 milliéquivalents de fonction tétraméthyl-2,2,6,6 pipéridinyle pour 100 g de polymère.

A titre indicatif les compositions polymériques stabilisées contiennent de 0,01 % à 5 % en poids de composé de formule (Ia) ou (Ib).

L'addition des composés de formule (Ia) ou (Ib) peut être effectuée pendant ou après la préparation des polymères.

Ces compositions de polymères organiques contenant les composés de formule (Ia) ou (Ib) peuvent contenir en outre les additifs et stabilisants habituellement utilisés tels que :

- les antioxydants comme les monophénols alkylés, les hydroquinones alkylées, les sulfures de diphényle hydroxylé, les alkylidène-bisphénols, les composés benzyliques, les acylaminophénols, les esters de l'acide (ditertiobutyl-3,5 hydroxy-4 phényl)-3 propionique, les esters de l'acide tertiobutyl-5 hydroxy-4 méthyl-3 phényl)-3 propionique, les esters de l'acide (dicyclohexyl-3,5 hydroxy-4 phényl)-3 propionique, les amides de l'acide (ditertiobutyl-3,5 hydroxy-4 phényl)-3 propionique ;
- les absorbeurs de rayons ultra-violets et stabilisants à la lumière comme les (hydroxy-2′ phényl)-2 benzotriazoles, les hydroxy-2 benzophénones, les esters d'acide benzoïque éventuellement substitués, les esters acryliques, les composés du nickel, les oxalamides ;
- les désactivants de métaux ;
- les phosphites et phosphonites ;
- les composés destructeurs de peroxydes ;
- les agents de nucléation ;
- les charges et agents de renforcement ;
- les plastifiants ;
- les lubrifiants ;
- les émulsionnants ;
- les pigments ;
- les azurants optiques ;
- les ignifugeants ;
- les antistatiques ;
- les porogènes.

Les compositions de polymères stabilisées peuvent être mises en oeuvre sous les formes les plus variées, par exemple sous forme d'objets moulés, de feuilles, de fibres, de matériaux cellulaires (masses), de profilés ou de produits de revêtement, ou comme feuillogènes (liants) pour peintures, vernis, colles ou ciments.

Les composés de formule (Ia) et (Ib) conviennent également comme stabilisants thermiques, notamment pour les polymères chlorés.

Particulièrement dans ces polymères, ils jouent donc à la fois le rôle de stabilisants UV et de stabilisants thermiques.

Ils peuvent être utilisés seuls ou associés à d'autres stabilisants thermiques comme par exemple les dérivés organiques de l'étain.

Les composés de formule (Ia) et (Ib) sont également souvent associés dans les polymères chlorés à des stabilisants thermiques primaires.

De préférence, ces stabilisants primaires sont des dérivés organiques du zinc, du calcium, du baryum, du magnésium et du strontium et le cas échant les hydrotalcites.

Un autre objet de l'invention consiste donc en des compositions à base de polymère chloré stabilisé, caractérisées en ce qu'elles contiennent :

a) une quantité efficace d'au moins un composé organique du zinc,

b) une quantité efficace d'au moins un composé organique du calcium, du baryum, du magnésium ou du strontium et/ou d'une hydrotalcite ;

c) une quantité efficace d'au moins un composé à fonction dihydro-1,4 pyridine de formule (Ia) ou (Ib).

Les polymères chlorés sont notamment le polychlorure de vinyle (PVC), le polychlorure de vinylidène ; les copolymères comportant majoritairement des motifs chlorure de vinyle obtenus à partir de chlorure de vinyle et d'autres monomères ; les mélanges de polymères ou copolymères dont une partie majoritaire est obtenue à partir de chlorure de vinyle.

De manière générale tout type de PVC convient, quel que soit son mode de préparation : polymérisation en masse, en suspension, en dispersion ou de tout autre type et quelle que soit sa viscosité intrinsèque.

Les homopolymères du chlorure de vinyle peuvent également être modifiés chimiquement, par exemple par chloration.

De nombreux copolymères du chlorure de vinyle peuvent également être stabilisés contre les effets de la chaleur et de la lumière, c'est-à-dire le jaunissement et la dégradation. Ce sont en particulier les copolymères obtenus par copolymérisation du chlorure de vinyle avec d'autres monomères présentant une liaison éthylénique polymérisable, comme par exemple l'acétate de vinyle ou le chlorure de vinylidène ; les acides maléique ou fumarique ou leurs esters ; les oléfines telles que l'éthylène, le propylène, l'hexène ; les esters acryliques ou méthacryliques ; le styrène ; les éthers vinyliques tels que le vinyldodécyléther.

Habituellement ces copolymères contiennent au moins 50 % en poids de motifs chlorure de vinyle et de préférence au moins 80 % en poids de motifs chlorure de vinyle.

Les compositions selon l'invention peuvent également contenir des mélanges à base de polymère chloré contenant des quantités minoritaires d'autres polymères, comme les polyoléfines halogénés ou les copolymères acrylonitrile-butadiène-styrène.

Le PVC seul ou en mélange avec d'autres polymères est le polymère chloré le plus largement utilisé dans les compositions de l'invention.

Les composés organiques du zinc sont de préférence les carboxylates et les phénolates de zinc.

Les plus couramment utilisés sont par exemple les sels de zinc des acides maléique, acétique, diacétique, propionique, hexanoïque, éthyl-2 hexanoïque, décanoïque, undécanoïque, laurique, myristique, palmitique, stéarique, oléïque, ricinoléïque, béhénique, hydroxystéarique, hydroxyundécanoïque, benzoïque, phénylacétique, paratertiobutylbenzoïque et salicylique ; les phénolates de zinc du phénol et des phénols substitués par un ou plusieurs radicaux alkyle, tels que les nonylphénols.

Pour des raisons pratiques ou pour des raisons économiques, on choisit de préférence parmi les composés organiques du zinc cités précédemment, le propionate de zinc, l'éthyl-2 hexanoate de zinc, le laurate de zinc, le stéarate de zinc, l'oléate de zinc, le ricinoléate de zinc, le benzoate de zinc, le paratertiobutylbenzoate de zinc, le salicylate de zinc, le maléate de zinc et de mono(éthyl-2 hexyle), les nonylphénates de zinc.

Généralement les composés organiques du zinc représentent de 0,005 % à 1 % en poids par rapport au polymère chloré, et de préférence de 0,01 % à 0,6 % en poids.

Les composés organiques du calcium, du baryum, du magnésium et du strontium sont de préférence les carboxylates et les phénolates de ces métaux.

Les plus couramment utilisés sont par exemple les sels de calcium, baryum, magnésium et strontium des acides maléique, acétique, diacétique, propionique, hexanoïque, éthyl-2 hexanoïque, décanoïque, undécanoïque, laurique, myristique, palmitique, stéarique, oléïque, ricinoléique, béhénique, hydroxystéarique, hydroxyundécanoïque, benzoïque, phénylacétique, paratertiobutylbenzoïque et salicylique ; les phénolates de calcium, baryum, magnésium et strontium du phénol et des phénols substitués par un ou plusieurs radicaux alkyle, tels que les nonylphénols.

Pour des raisons pratiques ou pour des raisons économiques, on choisit de préférence parmi les composés organiques de calcium, de baryum, de magnésium et de strontium cités précédemment les sels de calcium, de baryum et de magnésium des acides propionique, éthyl-2 hexanoïque, laurique, stéarique, oléïque, ricinoléïque, benzoïque, paratertiobutylbenzoïque, salicylique, du maléate de mono(éthyl-2 hexyle) ainsi que les nonylphénates de calcium, baryum et magnésium.

Généralement les composés organiques du calcium, du baryum, du magnésium et du strontium ou les hydrotalcites représentent de 0,005 % à 5 % en poids par rapport au polymère chloré et de préférence de 0,02 % à 2 % en poids.

Pour les applications alimentaires et notamment pour les bouteilles en PVC, on utilisera les composés organiques du calcium ou les mélanges composés organiques du calcium/composés organiques du magnésium.

Les hydrotalcites que l'on peut introduire dans les compositions à base de polymère chloré selon l'invention, à la place des composés organiques de calcium, de baryum, de magnésium et de strontium ou conjointement à ces composés, sont notamment les composés qui ont été décrits dans le brevet français FR-A-2 483 934 et dans le brevet européen EP-A-0 063 180.

Généralement les compositions à base de polymère chloré comprennent de 0,005 % à 5 % en poids de composé de formule (Ia) ou (Ib) par rapport au polymère chloré.

De préférence elles contiennent de 0,01 % à 2 % en poids de composé de formule (Ia) ou (Ib) par rapport au polymère chloré.

Par rapport aux dihydro-1,4 pyridines de l'art antérieur utlisées comme stabilisants thermiques des polymères chlorés, les composés de formule (I) présentent au moins la même efficacité au plan de la résistance au jaunissement pour un même poids, c'est-à-dire pour une plus faible quantité de motifs dihydro-1,4 pyridine, tout en ayant en outre une action efficace de protection contre les rayonnements UV.

Dans les compositions à base de polymère chloré de l'invention, il est naturellement possible d'introduire également d'autres additifs.

C'est ainsi notamment le cas des β-dicétones ou β-cétoaldéhydes qui ont une action synergique avec les composés à fonction dihydropyridine.

Ces β-dicétones ont été notamment décrites dans les brevets et certificats d'addition français publiés sous les numéros FR 2 292 227, FR 2 324 681, FR 2 351 149, FR 2 352 025, FR 2 383 988 et FR 2 456 132 et dans les brevets européens EP 0 040 286 et EP 0 046 161.

Comme exemples non limitatifs de telles β-dicétones, on peut citer le benzoylstéaroylméthane, le dibenzoylméthane, la benzoylacétone, le benzoyl méthyl-3 butanoylméthane, les méthoxycarbonylbenzoylbenzoylméthanes, les bis-β-dicétones telles que le bis(acétylacéto)-1,4 butane, le bis(benzoylacéto)-1,8 octane, le bis(acétylacéto)-1,4 benzène.

Lorsqu'elles sont présentes, les β-dicétones représentent de 0,005 % à 5 % en poids par rapport au polymère chloré et de préférence de 0,01 % à 2 % en poids.

Les compositions de l'invention peuvent comporter d'autres stabilisants thermiques secondaires tels que les polyols, les phosphites, les composés époxydés.

Les polyols ont généralement l'avantage d'allonger la durée de vie des polymères chlorés soumis à un traitement thermique.

Généralement il est préférable que les polyols utilisés aient un point d'ébullition supérieur à 150 ° C et de préférence supérieur à 170 ° C, à cause de la mise en oeuvre à température élevée des polymères chlorés.

A titre d'exemples de tels polyols on peut citer des triols comme le triméthylolpropane, le glycérol, l'hexanetriol-1,2,6, le butanetriol-1,2,4, le trishydroxyéthylisocyanurate ; des tétrols comme le pentaérythritol, le diglycérol ; des pentitols comme le xylitol, le tétraméthylolcyclohexanol ; des hexitols comme le mannitol, le sorbitol, le dipentaérythritol ; des polyols partiellement estérifiés par un acide carboxylique et dans la formule desquels au moins 3 fonctions hydroxyle sont libres ; des alcools polyvinyliques, notamment ceux dans lesquels il reste moins de 30 % en moles de groupes esters par rapport à l'ensemble de leurs groupes esters et hydroxyle et qui présentent une viscosité à 20 ° C en solution aqueuse à 4 % en poids comprise entre environ 4 x $10^{-3}$Pa.s et 60 x $10^{-3}$Pa.s.

Parmi ces polyols les préférés sont le xylitol, le mannitol, le sorbitol, le tétraméthylolcyclohexanol et les alcools polyvinyliques définis précédemment.

Lorsqu' il est présent dans les compositions selon l'invention, on utilise en général de 0,005 % à 1 % en poids de polyol par rapport au polymère chloré et de préférence de 0,01 % à 0,6 % en poids.

Les époxydes qui peuvent être utilisés dans les compositions selon l'invention sont généralement des composés complexes, habituellement des polyglycérides époxydés comme l'huile de soja époxydée qui est la plus fréquemment employée, l'huile de lin époxydée, les huiles de poisson époxydées, la talloil époxydée.

Les compositions selon l'invention peuvent comporter également des phosphites organiques, notamment des phosphites aliphatiques ou des phosphites aromatiques ou des phosphites mixtes aliphatiques et aromatiques.

Parmi les plus intéressants, on peut citer :

- les diphosphites de pentaérythrityle et de dialkyle,
- les diphosphites de pentaérythrityle et de diphényle,
- les diphosphites de pentaérythrityle et de bis(ditertiobutyl-2,4 phényle),
- les diphosphites de tétraalkyle et de bis(phénylène-1,4) diméthylméthane,
- les diphosphites de tétraalkyle et de bis(dialkyl-2,5 phénylène-1,4) alkylméthane,
- le diphosphite de diphényle, bis[(butoxy-2 éthoxy)-2 éthyle] et 4,4′-isopropylidènediphényle ;
- le diphosphite de tétrakis[(butoxy-2 éthoxy)-2 éthyle] et 4,4′-isopropylidènediphényle ;
- le triphosphite de diphényle, tris[(butoxy-2 éthoxy)-2 éthyle] et bis(4,4′-isopropylidènediphényle) ;
- le tétraphosphite de diphényle, tétrakis[(butoxy-2 éthoxy)-2 éthyle] et tris(4,4′-isopropylidènediphényle) ;
- le diphosphite de diphényle, bis[(butoxy-2 éthoxy)-2 éthyle] et 4,4′-isopropylidènediphényle ;
- le diphosphite de tétrakis[(butoxy-2 éthoxy)-2 éthyle] et 4,4′-isopropylidènediphényle ;
- le triphosphite de diphényle, tris[(butoxy-2 éthoxy)-2 éthyle] et bis(4,4′-isopropylidènediphényle) ;
- le tétraphosphite de diphényle, tétrakis[(butoxy-2 éthoxy)-2 éthyle] et tris(4,4′-isopropylidènediphényle) ;
- le pentaphosphite de diphényle, pentakis[(butoxy-2 éthoxy)-2 éthyle] et tétrakis(4,4′-isopropylidènediphényle) ;

- l'hexaphosphite de diphényle, hexakis[(butoxy-2 éthoxy)-2 éthyle] et pentakis(4,4'-isopropylidènediphényle) ;
- le triphosphite de pentakis[(butoxy-2 éthoxy)-2 éthyle] et bis(4,4'-isopropylidènediphényle) ;
- le tétraphosphite d'hexakis[(butoxy-2 éthoxy)-2 éthyle] et tris(4,4'-isopropylidènediphényle) ;
- le diphosphite de bis(ditertiobutyl-2,4 phényle), bis[(butoxy-2 éthoxy)-2 éthyle] et 4,4'-isopropylidènediphényle) ;
- le diphosphite de bis(ditertiobutyl-2,6 phényle), bis[(butoxy-2 éthoxy)-2 éthyle] et 4,4'-isopropylidènediphényle).

Lorsqu'il est présent, le phosphite représente généralement de 0,05 % à 5 % en poids par rapport au polymère chloré et de préférence de 0,1 % à 2 % en poids.

Les compositions selon l'invention peuvent également comporter des adjuvants habituels tels que des antioxydants phénoliques ; des agents anti-UV tels que les benzophénones ou les benzotriazoles.

Les compositions de l'invention peuvent être des formulations rigides, c'est-à-dire sans plastifiant ou semi-rigides, c'est-à-dire avec des teneurs en plastifiant réduites, telles que pour les applications dans le bâtiment ou pour la fabrication de bouteilles. Mais les compositions selon l'invention peuvent également être utilisées dans des formulations plastifiées telles que pour la fabrication de films à usage agricole.

De manière habituelle, l'incorporation des différents stabilisants ou adjuvants est faite sur le polymère chloré à l'état de poudre.

On peut bien entendu préparer un mélange de 2 ou plusieurs des composés constitutifs des compositions selon l'invention avant leur incorporation dans le polymère chloré.

Toutes les méthodes usuelles d'incorporation des différents stabilisants ou adjuvants dans le polymère peuvent être utilisées. Par exemple l'homogénéisation de la composition polymérique peut être réalisée sur malaxeur ou mélangeur à rouleaux, à une température telle que la composition devienne fluide, normalement entre 150 °C et 200 °C pour le PVC et pendant une durée suffisante, de l'ordre de quelques minutes à quelques dizaines de minutes.

les compositions de polymère chloré, et plus particulièrement de PVC, peuvent être mises en oeuvre selon toutes les techniques utilisées habituellement comme par exemple l'extrusion, l'injection, l'extrusion-soufflage, le calandrage ou le moulage par rotation.

Parmi les composés de formule (Ib), les composés de formule générale (VI) :

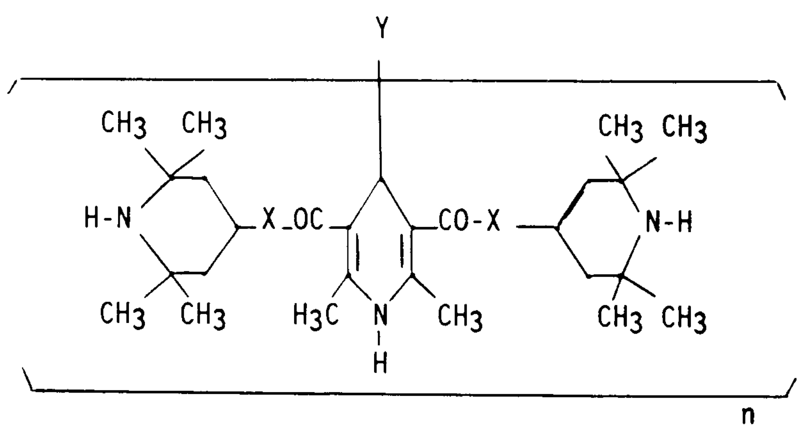

(VI)

dans laquelle :

- n est 1 ou 2
- X représente un atome d'oxygène ou un radical N-$R_2$ dans lequel $R_2$ représente un atome d'hydrogène ou un radical alkyle ayant 1 à 18 atomes de carbone ;
- Y représente un atome d'hydrogène, un radical alkyle ayant 1 à 18 atomes de carbone ou un radical phényle ;
- lorsque n = 2, Y représente un radical alkylène, linéaire ou ramifié, ayant 1 à 12 atomes de carbone.

sont particulièrement efficaces comme antioxydants dans les polymères organiques indiqués précédemment, c'est-à-dire plus particulièrement les polyoléfines, les polystyrènes, les polyalcadiènes, les polyuréthannes, les polyamides, les polyesters, les polycarbonates, les polysulfones, les polyéthers-sulfones, les polyéthers-cétones, les polymères acryliques, les polymères halogénés, les copolymères et les mélanges de ces polymères.

Parmi les polymères organiques précédents, le rôle d'antioxydant des composés de formule générale (VI) est plus particulièrement utile pour les polyoléfines comme le polyéthylène basse densité, le polyéthylène basse densité linéaire, le polyéthylène haute densité et le polypropylène, les polystyrènes, les polyalcadiènes, les polyamides, les polyesters et les polyuréthannes.

Généralement pour ce rôle d'antioxydant, on utilise de 0,01 % à 5 % en poids de composé de formule (VI) par rapport au poids de polymère à stabiliser et de préférence de 0,05 % à 2 % en poids par poids.

Il a été également remarqué que les composés de formule (VI) ont une action bénéfique au plan de ce que l'on appelle généralement le "process", c'est-à-dire essentiellement pendant la mise en forme à chaud du polymère.

On note que le polymère contenant un ou des composés de formule (VI) ne présente pas (ou peu) de dégradation ou de réticulation au cours de sa mise en forme à chaud, contrairement au polymère seul ou au polymère contenant soit un composé à groupement amine encombrée connu, soit un composé à fonction dihydropyridine connu, soit un mélange de ces 2 types de composés. C'est le cas notamment pour les polyoléfines et plus spécialement pour le polypropylène.

Les compositions polymériques contenant un ou plusieurs composés de formule (VI) peuvent bien évidemment contenir les adjuvants habituels à ce type de composition, comme cela a été indiqué précédemment.

En effet, hormis le choix plus spécifique des composés de formule (VI) lorsque l'on souhaite avoir des compositions polymériques plus résistantes à l'oxydation et plus stables lors de leur mise en forme, les compositions polymériques de l'invention comportent les mêmes types d'adjuvants, car pour être utilisable une composition doit être stabilisée contre les différents modes de dégradation : oxydation, chaleur, lumière ...

Dans le cadre de leur utilisation comme antioxydants, les composés de formule (VI) peuvent notamment être associés à certains phosphites organiques tels que particulièrement le phosphite de tris-(ditertiobutyl-2,4 phényle).

Les exemples qui suivent illustrent l'invention.

EXEMPLE 1 - Préparation de la diméthyl-2,6 bis[(tétraméthyl-2,2,6,6 pipéridinyl-4) oxycarbonyl]-3,5 dihydro-1,4 pyridine

1a - Préparation de l'acétoacétoxy-4 tétraméthyl-2,2,6,6 pipéridine

Dans un ballon tricol de 500 $cm^3$, équipé d'une agitation centrale, d'une gaine thermométrique, d'un réfrigérant ascendant et d'une ampoule de coulée, on charge :

- 31,4 g (0,2 mol) d'hydroxy-4 tétraméthyl-2,2,6,6 pipéridine,
- 200 $cm^3$ de toluène
- 1 $cm^3$ de triéthylamine (catalyseur).

On chauffe à 70 °C sous agitation, puis on coule régulièrement en 30 min 16,8 g (0,2 mol) de dicétène, tout en maintenant la température à 70 °C.

On maintient cette température pendant encore 2h 30 min après la fin de la coulée.

L'ensemble de ces opérations est effectué sous atmosphère d'azote.

On élimine ensuite le toluène, le triéthylamine et les traces de dicétène sous pression réduite par chauffage progressif :

- pression de 2000 Pa diminuée progressivement jusqu'à 65 Pa
- température de 20 °C augmentée jusqu'à 65 °C en fin d'opération.

On obtient ainsi 48,1 g d'une huile jaune orangé, homogène, titrant par acidimétrie 393 milliéquivalents (meq) pour 100 g en fonction $\beta$-céto-ester (théorie de 414,9 meq/100 g), ce qui correspond à une pureté d'environ 95 %.

La structure attendue est confirmée par son spectre infra-rouge et son spectre de masse.

1b - Préparation de la diméthyl-2,6 bis[(tétraméthyl-2,2,6,6 pipéridinyl-4)oxycarbonyl-3,5 dihydro-1,4 pyridine

Dans l'appareillage décrit dans l'exemple 1a, on charge :

| | |
|---|---|
| - acétoacétoxy-4 tétraméthyl-2,2,6,6 pipéridine préparée en 1a (à 95 %)<br>- hexaméthylène tétramine<br>- acétate d'ammonium<br>- isopropanol à 20 % d'eau en volume | 38,05 g (0,15 mol)<br>2,00 g (0,014 mol)<br>4,95 g (0,0643 mol)<br>100 $cm^3$ |

On agite et on chaufe le mélange réactionnel homogène vers 72°C, sous atmosphère d'azote pendant 2h 50 min.

Après refroidissement, on ajuste le pH du milieu à 11,4, par addition d'une solution 5N de soude. On ajoute alors 700 $cm^3$ d'eau sous forte agitation ; il se forme un précipité que l'on essore, lave à l'eau et sèche à 80°C sous pression réduite.

On obtient ainsi 26,8 g d'un solide jaune ayant un point de fusion de 189 - 190°C pratiquement pur et dont les spectres infra-rouge et masse sont en accord avec la structure attendue.

Le rendement en produit pur isolé est de 75 % par rapport au composé 1a engagé.

EXEMPLE 2 -

2a - Préparation d'acétoacétoxy-4 pentaméthyl-1,2,2,6,6 pipéridine

On répète l'exemple 1a en mettant en oeuvre de l'hydroxy-4 tétraméthyl-2,2,6,6 N-méthyl pipéridine.

On obtient une huile jaune orangé titrant par acidimétrie 369 meq/100 g (théorie de 392 meq/100 g) : pureté de 94 % environ.

2b - Préparation de diméthyl-2,6 bis[(pentaméthyl-1,2,2,6,6 pipéridinyl-4) oxycarbonyl)-3,5 dihydro-1,4 pyridine

On répète l'exemple 1b en remplaçant l'acétoacétoxy-4 tétraméthyl-2,2,6,6 pipéridine par la même quantité molaire d'acétoacétoxy-4 pentaméthyl-1,2,2,6,6 pipéridine obtenue en 2a.

On obtient la diméthyl-2,6 bis[(pentaméthyl-1,2,2,6,6 pipéridinyl-4) oxycarbonyl]-3,5 dihydro-1,4 pyridine sous forme d'un solide jaune ayant un point de fusion de 232°C et dont les spectres infra-rouge, de résonance magnétique nucléaire (RMN) et de masse sont en accord avec la stucture.

EXEMPLE 3

3a - Préparation de l'acétoacétamido-4 tétraméthyl-2,2,6,6 pipéridine

On répète l'exemple 1a en mettant en oeuvre l'amino-4 tétraméthyl-2,2,6,6 pipéridine, sans engager de catalyseur et en réalisant la coulée du dicétène à une température de 0°C à 10°C ; on obtient l'acétoacétamido-4 tétraméthyl-2,2,6,6 pipéridine sous la forme d'un solide blanc ayant un point de fusion de 115°C.

Le rendement en produit isolé pur par rapport à l'amino-4 tétraméthyl-2,2,6,6 engagé est de 90 %.

3b - Préparation de la diméthyl-2,6 bis[(tétraméthyl-2,2,6,6 pipéridinyl-4) aminocarbonyl]-3,5 dihydro-1,4 pyridine

Dans un erlenmeyer de 100 $cm^3$, on charge :

- 1,50 g (0,050 mol) de formaldéhyde,
- 21,3 g (0,089 mol) d'acétoacétamido-4 tétraméthyl-2,2,6,6 pipéridine préparé en 3a,
- 20 $cm^3$ d'éthanol absolu,
- 5 gouttes de diéthylamine.

Le mélange réactionnel homogène ainsi obtenu est laissé au repos pendant 24 heures à 4°C, puis pendant 48 h à température ambiante.

On rajoute ensuite 4,6 $cm^3$ d'une solution aqueuse ammoniacale contenant 290 g/litre de $NH_3$ (soit 0,078 mol de $NH_3$).

On chauffe à 75 - 80°C pendant 5 heures, puis on laisse pendant 24 heures à température ambiante.

Après distillation des solvants sous pression réduite, on obtient 23 g d'un solide orangé légèrement pâteux.

Ce solide est dissous danc 200 $cm^3$ de méthanol, puis précipité par addition de 500 $cm^3$ d'eau sous forte agitation.

Le précipité obtenu est filtré, lavé à l'eau et séché à 40 ° C sous pression réduite.

On obtient ainsi 4,5 g d'un solide jaune ayant un point de fusion de 170 ° C et dont la structure de diméthyl-2,6 bis[(tétraméthyl-2,2,6,6 pipéridinyl-4) aminocarbonyl]-3,5 dihydro-1,4 pyridine est confirmée par RMN.

EXEMPLE 4

4a - Préparation de la N-n.butylacétoacétamido-4 tétraméthyl-2,2,6,6 pipéridine

En opérant comme dans l'exemple 3a, mais en mettant en oeuvre la n.butylamino-4 tétraméthyl-2,2,6,6 pipéridine, on obtient la N-n.butylacétoacétamido-4 tétraméthyl-2,2,6,6 pipéridine sous la forme d'un liquide jaune, visqueux, de pureté supérieure à 95 %.

4b - Préparation de la diméthyl-2,6 bis[(tétraméthyl-2,2,6,6 pipéridinyl-4) n.butylaminocarbonyl]-3,5 dihydro-1,4 pyridine

On répète l'exemple 3b en utilisant le composé préparé en 4a à la place de l'acétoacétamido-4 tétraméthyl-2,2,6,6 pipéridine.

On obtient la diméthyl-2,6 bis[(tétraméthyl-2,2,6,6 pipéridinyl-4) n.butylaminocarbonyl]-3,5 dihydro-1,4 pyridine sous la forme d'un solide blanc ayant un point de fusion de 172 ° C. Les spectres infra-rouge et de masse sont en accord avec la structure attendue.

EXEMPLE 5 - Préparation du bis[diméthyl-2,6 bis[pentaméthyl-1,2,2,6,6 pipéridinyl-4 oxycarbonyl]-3,5 dihydro-1,4 pyridinyl-4]-1,6 hexane

On répète l'exemple 3b en remplaçant le formaldéhyde par 3,55 g (0,025 mol) d'octanedial et en engageant l'acétoacétoxy-4-pentaméthyl-1,2,2,6,6 pipéridine préparée dans l'exemple 2a.

On obtient (avec un rendement de 70 %), 19,7 g d'un solide jaune ayant un point de fusion d'environ 110 ° C et dont la structure est confirmée par RMN.

EXEMPLES 6 à 11 et essais comparatifs A1 et A2 -
Stabilisation thermique du PVC

On prépare la composition de base A suivante :

| | |
|---|---|
| - PVC en poudre préparé par polymérisation ou suspension et commercialisé sous la marque LACQVYL SO 71 S (indice de viscosité selon la norme NF T 51013:80) | 1.000 g |
| - renforçateur de choc (copolymère butadiène/styrène/méthacrylate de méthyle) | 80g |
| - lubrifiant à base d'ester de colophane (cire E) | 5 g |
| - huile de soja époxydée | 30 g |
| - stéarate de calcium | 3, g |
| - stéarate de zinc | 2,5 g |

Après homogénéisation en mélangeur rapide à froid, on prélève 8 fractions de cette composition A. A chaque fraction on ajoute une quantité de composé préparé dans l'exemple 2b (DHP/HALS ex 2b), dans l'exemple 1b (DHP/HALS ex 1b), dans l'exemple 5 (DHP/HALS ex 5), ou de diméthyl-2,6 bis-(dodécyloxycarbonyl)-3,5 dihydro-1,4 pyridine (DHP art antérieur) et/ou de stéaroylbenzoylméthane (SBM). Les quantités en poids pour 100 g de PVC sont indiquées dans le tableau I ci-après.

On prépare à l'aide des différentes compositions ainsi obtenues, ainsi qu'avec la composition A non modifiée, des feuilles de 1 mm d'épaisseur par malaxage sur un mélangeur à 2 cylindres pendant 3 min à 180 ° C.

A partir d'éprouvettes (environ 1 cm x 2 cm) découpées dans ces feuilles, on effectue un test de vieillissement thermique en étuve ventilée à 180 ° C et l'on suit en fonction du temps l'évolution de la coloration GARDNER;

Le tableau I rassemble les indices de coloration GARDNER mesurés pour différentes durées de vieillissement jusqu'à 30 minutes.

| ESSAIS | STABILISANTS | | INDICES GARDNER EN FONCTION DU TEMPS EN MINUTES | | | | |
|---|---|---|---|---|---|---|---|
| | NATURE | Poids en g/100 g PVC | 0 | 7 | 14 | 21 | 30 |
| Témoin | Néant | --- | 3 | 7 | 7 | 7 | 7 |
| Essai A1 | DHP/art antérieur | 0,2 | 1 | 1,5 | 1,5 | 1,5 | 2,5 |
| Exemple 6 | DHP/HALS ex 2b | 0,2 | 0,5 | 1 | 1 | 1 | 2 |
| Exemple 7 | DHP/HALS ex 2b | 0,1 | 1 | 2 | 2 | 2 | 3 |
| Exemple 8 | DHP/HALS ex 2b | 0,3 | 0,5 | 1 | 1 | 1 | 2 |
| Essai A2 | SBM | 0,3 | 0 | 0 | 1 | 1,5 | 2 |
| Exemple 9 | DHP/HALS ex 2b<br>SBM | 0,1<br>0,2 | 0 | 0 | 0 | 0,5 | 1,5 |
| Essai A3 | SBM<br>DHP/art antérieur | 0,2<br>0,1 | 0 | 0 | 0 | 1 | 2 |
| Exemple 10 | DHP/HALS ex 1b | 0,2 | 0,5 | 1 | 1 | 1 | 2 |
| Exemple 11 | DHP/HALS ex 5 | 0,2 | 1 | 1,5 | 2 | 2 | 6 |

TABLEAU I

EXEMPLE 12 et essai comparatif B1 -

Stabilisation UV du PVC

On prépare la composition de base B suivante :

| | |
|---|---|
| - PVC LACQVYL SO 71 S | 1.000 g |
| - lubrifiant interne (mélange d'hexadécanol et d'octadécanol) | 14 g |
| - lubrifiant cire E (à base d'ester de colophane) | 2 g |
| - lubrifiant cire OP (à base de montanate de propylèneglycol partiellement saponifié) | 3 g |
| - stabilisant thioétain * | 15 g |

(*mélange de 75 % en poids de dioctylstanniobis(sulfuroacétate d'isooctyle), et de 25 % en poids de trioctylstanniosulfuroacétate d'isooctyle)

Après homogénéisation en mélangeur à froid, on prélève 2 fractions de cette composition B. A chaque fraction, on ajoute soit 0,10 g (pour 100 g de PVC) de composé de formule (Ia) ou (Ib) préparé dans l'exemple 1b (DHP/HALS ex 1b), soit 0,10 g (pour 100 g de PVC) d'un anti-UV généralement utilisé dans le PVC (anti UVP).

A l'aide de ces différentes compositions ainsi qu'avec la composition B seule, on prépare des feuilles d'environ 1 mm d'épaisseur par malaxage sur un mélangeur à 2 cylindres pendant 3 min à 180 °C.

A partir de ces feuilles, on prépare des films de 200 µm à l'aide d'une presse à plateaux à 185 °C.

Ces films sont placés dans une enceinte de vieillissement accéléré artificiel, à 30 °C, équipée d'un tube fluorescent de type B 40 W émettant entre 275 nm et 380 nm, avec un maximum d'intensité à 310 nm.

On suit dans le spectre visible l'évolution de la densité optique des séquences polyéniques (contenant 10 doubles liaisons conjuguées) à une longueur d'onde λ = 447 nm.

Après 750 heures de vieillissement on obtient les valeurs indiquées dans le tableau (II) ci-après.

| Essais | Stabilisants anti UV en g/100 g PVC | Densité optique à θ = 447 nm après 750 heures de vieillissement |
|---|---|---|
| Témoin | néant | 0,18 |
| Exemple 12 | DHP/HALS ex 1b | 0,04 |
| Essai B1 | Anti UVP * | 0,07 |

TABLEAU II

* Anti UVP = hydroxy-2 octyloxy-4 benzophénone (stabilisant anti UV de type benzophénone très largement utilisé dans le PVC)

On remarque l'action anti UV très nette du composé de formule (Ia) et (Ib) préparé dans l'exemple 1b ; cette efficacité est supérieure à celle d'un anti UV de type benzophénone très largement utilisé. En outre cette efficacité existe dans une composition stabilisée thermiquement par un composé thioétain, dont l'action photosensibilisatrice est connue et importante.

EXEMPLE 13 - Photostabilisation du polypropylène (PP) APPRYL 3030 P commercialisé par BP Chimie

Dans un mélangeur lent, on prépare environ 300 g de chacun des mélanges dont la composition pondérale est indiquée dans le tableau (III) suivant :

| Composition | C | D | E | F | G | H | J | K | L | M |
|---|---|---|---|---|---|---|---|---|---|---|
| PP | 100 | 100 | 100 | 100 | 100 | 100 | 100 | 100 | 100 | 100 |
| Stéarate de Ca | 0,1 | 0,1 | 0,1 | 0,1 | 0,1 | 0,1 | 0,1 | 0,1 | 0,1 | 0,1 |
| antioxydant phénolique* | 0,02 | 0,02 | 0,02 | 0,02 | 0,02 | 0,02 | 0,02 | 0,02 | 0,02 | 0,02 |
| anti UV commercial** CHIMASSORB944 | 0 | 0 | 0,15 | 0 | 0 | 0 | 0 | 0,30 | 0 | 0 |
| HALS commerciale*** TINUVIN 770 | 0 | 0,15 | 0 | 0 | 0 | 0 | 0,30 | 0 | 0 | 0 |
| HALS/DHP Ex 1b | 0 | 0 | 0 | 0,15 | 0 | 0 | 0 | 0 | 0,30 | 0 |
| HALS/DHP Ex 2b | 0 | 0 | 0 | 0 | 0,15 | 0 | 0 | 0 | 0 | 0,30 |
| HALS/DHP Ex 5 | 0 | 0 | 0 | 0 | 0 | 0,15 | 0 | 0 | 0 | 0 |

TABLEAU III

* tétra(hydroxy-4 ditertiobutyl-3,5 phényl)-3 propionate de pentaérythrityle

** CHIMASSORB 944

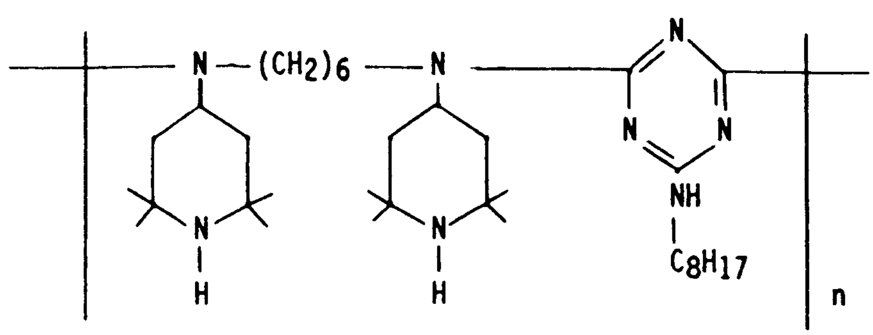

n > 1

*** TINUVIN 770 : décanedioate de bis(tétraméthyl-2,2,6,6-pipéridinyle-4)

Ces compositions sont extrudées dans les conditions suivantes :

- extrudeuse de marque THORET : diamètre de la vis = 20 mm longueur de la vis = 400 mm
- profil de température :
  - . zone 1 = 200 ° C
  - . zone 2 = 220 ° C
  - . zone 3 = 220 ° C
  - . zone 4 = 230 ° C
  - . tête de filière = 215 ° C

Le jonc obtenu est granulé, puis les granulés sont pressés en films de 200 µm à l'aide d'une presse CARVER dans les conditions suivantes :

| | |
|---|---|
| - température | = 210 ° C |
| - durée | = 5 min |
| - pression | = 20 MPa. |

Ces films sont exposés dans une enceinte de vieillissement accéléré de type SAIREM-SEPAP 12-24. Dans cette enceinte les échantillons sont disposés sur une tourelle cylindrique animée d'un mouvement de rotation circulaire. La tourelle est elle-même située au centre d'une enceinte parallélépipédique dont les 4 angles sont occupés par une lampe à vapeur de mercure ("moyenne pression" de type MAZDA MA 400 W.

L'enveloppe de la lampe ne laisse passer que les radiations de longueur d'onde supérieure à 300 nm (un tel dispositif est décrit dans le brevet français 2 430 609).

La température de l'enceinte est maintenue à 60 ° C par un système de régulation.

Le vieillissement des films est suivi par spectrométrie infra-rouge : la densité optique de la bande carbonyle à 1720-1740 $cm^{-1}$ traduit le degré de photooxydation du matériau polymérique.

Les résultats obtenus sont rassemblés dans le tableau IV ci-après.

TABLEAU IV

| Composition | Stabilisant anti-UV | Durée pour obtenir une densité optique de 0,3 |
|---|---|---|
| C | néant | 35 h |
| D | TINUVIN 770 | 320 h |
| E | CHIMMASORB 944 | 232 h |
| F | HALS/DHP Ex. 1b | 250 h |
| G | HALS/DHP Ex. 2b | 270 h |
| H | HALS/DHP Ex. 5 | 234 h |
| J | TINUVIN 770 | 600 h |
| K | CHIMMASORB 944 | 400 h |
| L | HALS/DHP Ex. 1b | 450 h |
| M | HALS/DHP Ex. 2b | 400 h |

EXEMPLE 14 - Photostabilisation du polypropylène

On prépare séparément 5 mélanges de poudres suivantes :

| | |
|---|---|
| - polypropylène NESTE | 100 g |
| - antioxydant phénolique IRGANOX 1076 * | 0,05 g |
| - stéarate de calcium | 0,10 g |
| - anti-UV à tester | 0 ou 0,15 (voir tableau V) |

* IRGANOX 1076 = (hydroxy-4 ditertiobutyl-3,5 phényl)-3 propionate d'octadécyle.

On homogénéise à sec un mélangeur lent pendant 5 minutes ces différents mélanges.

On prépare ensuite des feuilles par passage pendant 5 min à 170 °C dans un mélangeur à 2 cylindres.

A partir de ces feuilles, on prépare des films de 200 µm d'épaisseur par passage sous presse SCHABENTANN dans les conditions suivantes :

| | |
|---|---|
| - contact | 2 min à 220 °C |
| - pressage | 1 min sous 37 MPa (370 bars) |

Les films obtenus sont ensuite placés dans une enceinte artificielle de vieillissement équipé de tubes fluorescents du type B 400 W : le spectre est compris entre 275 et 380 nm avec un maximum à 310 nm. La température de l'enceinte est maintenue à 55 °C en atmosphère saturée d'humidité.

On suit comme dans l'exemple précédent l'évolution de la densité optique de la bande carbonyle à 1720 - 1740 $cm^{-1}$ qui traduit le degré de vieillissement du polymère.

On a obtenu les résultats suivants (durée de vieillissement nécessaire pour obtenir une densité optique de 0,3).

TABLEAU V

| Stabilisant anti-UV | | Durée pour obtenir une densité optique de 0,3 |
|---|---|---|
| Nature | Quantité g | |
| Néant | 0 | 40 h |
| CHIMASSORB 944 | 0,15 | 270 h |
| HALS/DHP ex. 1b | 0,15 | 400 h |
| HALS/DHP ex. 3b | 0,15 | 310 h |
| HALS/DHP ex. 4b | 0,15 | 300 h |

EXEMPLE 15 - Stabilisation thermomécanique du polypropylène (PP) VB 65 11 B commercialisé par la société Neste .

Dans un mélangeur rapide, on prépare chacun des mélanges dont la composition pondérale est indiquée dans le tableau (VI) suivant :

| Composition | N | P | Q | R | S | T | U |
|---|---|---|---|---|---|---|---|
| PP | 100 | 100 | 100 | 100 | 100 | 100 | 100 |
| Stéarate de Ca | 0,1 | 0,1 | 0,1 | 0,1 | 0,1 | 0,1 | 0,1 |
| antioxydant IRGANOX 1076* | 0,05 | 0,05 | 0,05 | 0,05 | 0,05 | 0,05 | 0,05 |
| HALS commercial** TINUVIN 622 | 0 | 0 | 0,2 | 0 | 0 | 0,2 | 0 |
| HALS commerciale*** TINUVIN 770 | 0 | 0,2 | 0 | 0 | 0,2 | 0 | 0 |
| DHP **** art antérieur | 0 | 0 | 0 | 0,2 | 0,1 | 0,1 | 0 |
| HALS/DHP Ex 1b | 0 | 0 | 0 | 0 | 0 | 0 | 0,1 |

TABLEAU VI

* IRGANOX 1076 = (hydroxy-4 ditertiobutyl-3,5 phényl)-3 propionate d'octadécyle.

** TINUVIN 622 =

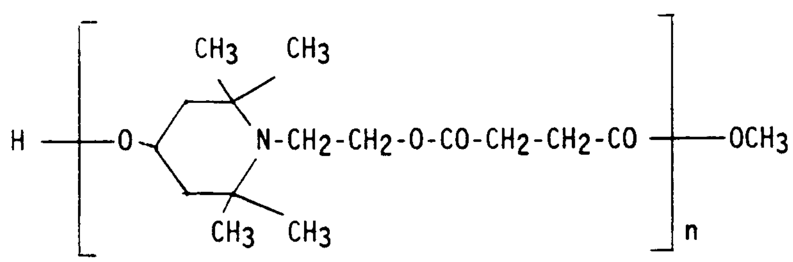

n > 1

*** TINUVIN 770 = voir exemple 13.

**** DHP art antérieur = voir exemples 6 à 11.

La stabilisation du polymère fondu est examinée à l'aide d'un plastographe Brabender. La température de la cuve de malaxage est maintenue à 220 °C.

Le couple exercé sur le mélange est suivi au cours du temps.

On évalue la stabilité thermomécanique par le rapport du couple exercé sur chacun des mélanges N à U et du couple exercé sur le mélange N, après 15 min d'application de la contrainte thermomécanique.

Plus ce rapport est élevé, meilleure est la stabilité thermomécanique.

Le tableau suivant VII donne les valeurs de stabilité pour les différentes compositions.

TABLEAU VII

| Composition | Stabilisant | Stabilité thermomécanique |
|---|---|---|
| N | néant | 1 |
| P | TINUVIN 770 | 0,76 |
| Q | TINUVIN 622 | 0,95 |
| R | DHP art antérieur | 1,23 |
| S | TINUVIN 770 + DHP | 0,95 |
| T | TINUVIN 662 + DHP | 1,1 |
| U | HALS/DHP Ex. 1b | 2,18 |

EXEMPLE 16 - Stabilisation thermomécanique du polypropylène (PP) VB 65 11 B commercialisé par la société Neste .

La tenue thermomécanique des compositions indiqué dans le tableau VIII ci-après est mesurée dans les conditions de l'exemple 15.

TABLEAU VIII

| Composition | a | b | c | d | e | f | g | h | j | k | l | m | n |
|---|---|---|---|---|---|---|---|---|---|---|---|---|---|
| PP | 100 | 100 | 100 | 100 | 100 | 100 | 100 | 100 | 100 | 100 | 100 | 100 | 100 |
| Stéarate de Ca | 0,1 | 0,1 | 0,1 | 0,1 | 0,1 | 0,1 | 0,1 | 0,1 | 0,1 | 0,1 | 0,1 | 0,1 | 0,1 |
| antioxydant IRGANOX 1010* | 0 | 0,05 | 0 | 0 | 0,1 | 0 | 0 | 0,05 | 0,1 | 0,1 | 0 | 0 | 0 |
| phosphite GARBEFIX OS 240 ** | 0 | 0 | 0,1 | 0 | 0 | 0,2 | 0 | 0,1 | 0,1 | 0,2 | 0,1 | 0,1 | 0,2 |
| HALS/DHP Ex 1b | 0 | 0 | 0 | 0,05 | 0 | 0 | 0,1 | 0 | 0 | 0 | 0,05 | 0,1 | 0,1 |

* IRGANOX 1010 = tétra(hydroxy-4 ditertiobutyl-3,5 phényl)-3 propionate de pentaérythrityle

** GARBEFIX OS 240 = phosphite de tris(ditertiobutyl-2,4 phényle)

Le tableau IX suivant donne les valeurs de stabilité thermomécanique mesurées pour les différentes compositions.

TABLEAU IX

| Composition | Stabilisants | Stabilité thermomécanique |
|---|---|---|
| a | néant | 1 |
| b | IRGANOX 1010 | 1,1 |
| c | GARBEFIX OS 240 | 1,7 |
| d | HALS/DHP Ex. 1b | 1,3 |
| e | IRGANOX 1010 | 1,6 |
| f | GARBEFIX OS 240 | 1,9 |
| g | HALS/DHP Ex. 1b | 2,2 |
| h | IRGANOX 1010 + GARBEFIX OS 240 | 1,6 |
| j | IRGANOX 1010 + GARBEFIX OS 240 | 2,4 |
| k | IRGANOX 1010 + GARBEFIX OS 240 | 2,5 |
| l | HALS/DHP Ex. 1b + GARBEFIX OS 240 | 2,3 |
| m | HALS/DHP Ex. 1b + GARBEFIX OS 240 | 2,3 |
| n | HALS/DHP Ex. 1b + GARBEFIX OS 240 | 2,4 |

EXEMPLE 17 - Stabilisation thermique du polypropylène (PP) VB 6511 B commercialisé par la société Neste .

On prépare séparément 4 mélanges de compositions suivantes :

| | |
|---|---|
| - polypropylène | 100 g |
| - antioxydant IRGANOX 1076 | 0,05 g |
| - stéarate de calcium | 0,1 g |
| - stabilisant (voir tableau X) | 0 ou 0,15 g |

On homogénéise dans un mélangeur rapide pendant 5 min chacun de ces mélanges.

On prépare ensuite des feuilles par malaxage pendant 5 min dans un mélangeur à cylindres à la température de 170 °C.

Les feuilles obtenues sont ensuite pressées sous une presse SCHWABENTHAM dans les conditions suivantes :

| | |
|---|---|
| - contact | 40 s à 220 °C |
| - pressage | 2 min sous 2,1 MPa |

Les plaques ainsi obtenues ont une épaisseur de 1 mm.

Ces plaques sont ensuite placées dans une étuve ventilée à la température de 150 °C.

La dégradation thermique du polymère est déterminée par l'apparition de microfissures provoquant une fragilisation du polymère.

TABLEAU X

| Stabilisant | Temps de dégradation |
|---|---|
| néant | 280 h |
| TINUVIN 770 | 475 h |
| HALS/DHP Ex. 1b | 475 h |
| HALS/DHP Ex. 3b | > 700 h |

## Revendications

**Revendications pour les Etats contractants suivants : AT, BE, CH, DE, DK, FR, GB, GR, IT, LI, LU, NL, SE**

**1.** Composés à fonction dihydro-1,4-pyridine de formules générales (Ia) et (Ib):

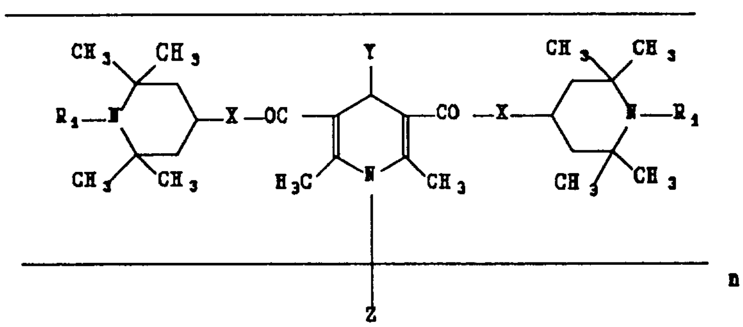

(Ia)

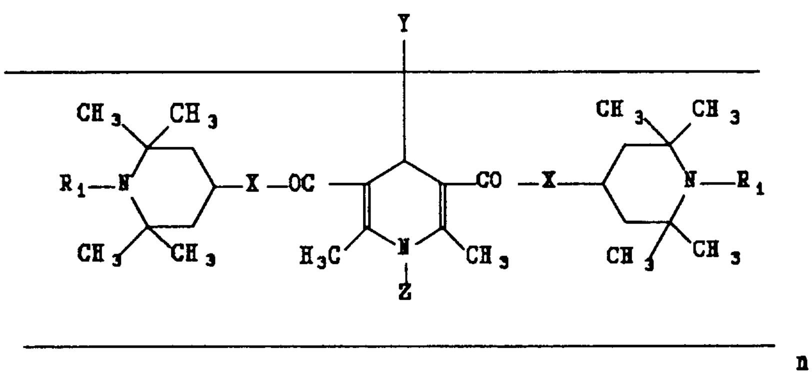

(Ib)

dans lesquelles:

- n est 1 ou 2,
- $R_1$ représente un atome d'hydrogène, un radical méthyle ou un radical acétyle,
- X représente un atome d'oxygène ou un radical N-$R_2$ dans lequel $R_2$ représente un atome d'hydrogène ou un radical alkyle ayant 1 à 18 atomes de carbone,
- Y représente un atome d'hydrogène, un radical alkyle ayant 1 à 18 atomes de carbone ou un radical phényle,
- Z représente un atome d'hydrogène ou un radical alkyle ayant 1 à 18 atomes de carbone,
- lorsque n = 2, l'un des deux symboles Y et Z peut représenter un radical alkylène ayant 1 à 12 atomes de carbone.

2. Composés selon la revendication 1, caractérisés par le fait qu'ils sont choisis dans le groupe comprenant:
   - la diméthyl-2,6-bis[(pentaméthyl-1,2,2,6,6-pipéridinyl-4)-oxycarbonyl]-3,5-dihydro-1,4-pyridine;
   - la diméthyl-2,6-bis[(tétraméthyl-2,2,6,6-pipéridinyl-4)-oxycarbonyl]-3,5-dihydro-1,4-pyridine;
   - la diméthyl-2,6-bis[(tétraméthyl-2,2,6,6-pipéridinyl-4)-aminocarbonyl]-3,5-dihydro-1,4-pyridine;
   - la diméthyl-2,6-bis[(tétraméthyl-2,2,6,6-pipéridinyl-4)-N-butylaminocarbonyl]-3,5-dihydro-1,4-pyridine;
   - la bis[diméthyl-2,6-bis[(pentaméthyl-1,2,2,6,6-pipéridinyl-4)-oxycarbonyl]-3,5-dihydro-1,4-pyridinyl-4]-1,6-hexane.

3. Procédé de préparation des composés de formules (Ia) et (IB) selon l'une des revendications 1 et 2, caractérisé en ce que l'on fait réagir un ester acétoacétique ou un acétoacétamide de formule (II)

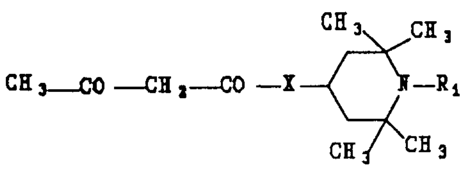

(II)

avec un aldéhyde de formule (III) $Y\text{-}(\text{-}CHO)_n$ et une amine de formule (IV) $Z\text{-}(\text{-}NH_2)n$,
les différents symboles n, X et $R_1$, Y et Z dans les formules (II), (III) et (IV) ayant les significations indiquées dans la revendication 1 pour les formules (Ia) et (Ib).

4. Compositions de polymère organique stabilisé contre les effets néfastes de la lumière et des rayons ultra-violets par une quantité efficace d'au moins un composé de formule (Ia) ou (Ib) selon l'une des revendications 1 ou 2.

5. Compositions selon la revendication 4, caractérisées en ce que le polymère organique est choisi parmi les polyoléfines, les polystyrènes, les polyalcadiènes, les polyuréthanes, les polyamides, les polyesters, les polycarbonates, les polysulfones, les polyéthers-sulfones, les polyéthers-cétones, les polymères acryliques, les polymères halogénés, leurs copolymères et leurs mélanges.

6. Compositions selon l'une des revendications 4 et 5, caractérisées en ce qu'elles contiennent de 0,004 à 20 milliéquivalents de fonction tétraméthyl-2,2,6,6-pipéridinyle pour 100 g de polymère.

7. Compositions selon la revendication 6, caractérisées en ce qu'elles contiennent de 0,02 à 4 milliéquivalents de fonction tétraméthyl-2,2,6,6-pipéridinyle pour 100 g de polymère.

8. Utilisation des composés de formules (Ia) et (Ib) selon l'une des revendications 1 et 2 comme stabilisants thermiques et stabilisants UV dans les polymères chlorés.

9. Compositions à base de polymère chloré stabilisé selon la revendication 8, caractérisées en ce qu'elles contiennent:
   a) une quantité efficace d'au moins un composé organique du zinc,
   b) une quantité efficace d'au moins un composé organique du calcium, du baryum, du magnésium ou du strontium et/ou d'une hydrotalcite,
   c) une quantité efficace d'au moins un composé à fonction dihydro-1,4-pyridine de formule (Ia) ou (Ib).

10. Compositions selon la revendication 9, caractérisées en ce qu'elles comprennent de 0,005% à 5% en poids de composé de formule (Ia) ou (Ib) par rapport au polymère chloré.

11. Compositions selon la revendication 10, caractérisées en ce qu'elles comprennent de 0,01% à 2% en poids de composé de formule (Ia) ou (Ib) par rapport au polymère chloré.

12. Compositions selon l'une des revendications 9 à 11, caractérisées en ce qu'elles comprennent:

- de 0,005% à 1% en poids d'au moins un composé organique du zinc par rapport au polymère chloré,
- de 0,005% à 5% en poids d'au moins un composé organique du calcium, du baryum, du magnésium et du strontium ou une hydrotalcite par rapport au polymère chloré.

**13.** Compositions selon la revendication 12, caractérisées en ce qu'elles comprennent:
- de 0,005% à 1% en poids d'au moins un composé organique du zinc par rapport au polymère chloré,
- de 0,02% à 2% en poids d'au moins un composé organique du calcium, du baryum, du magnésium et du strontium ou une hydrotalcite par rapport au polymère chloré.

**14.** Compositions selon la revendication 12, caractérisées en ce qu'elles comprennent:
- de 0,01% à 0,6% en poids d'au moins un composé organique du zinc par rapport au polymère chloré,
- de 0,005% à 5% en poids d'au moins un composé organique du calcium, du baryum, du magnésium et du strontium ou une hydrotalcite par rapport au polymère chloré.

**15.** Compositions selon l'une des revendication 13 et 14, caractérisées en ce qu'elles comprennent:
- de 0,01% à 0,6% en poids d'au moins un composé organique du zinc par rapport au polymère chloré,
- de 0,02% à 2% en poids d'au moins un composé organique du calcium, du baryum, du magnésium et du strontium ou une hydrotalcite par rapport au polymère chloré.

**16.** Compositions selon l'une des revendications 9 à 15, caractérisées en ce qu'elles comprennent de 0,005% à 5% en poids d'au moins une $\beta$-dicétone par rapport au polymère chloré.

**17.** Compositions selon la revendication 16, caractérisées en ce qu'elles comprennent de 0,01% à 2% en poids d'au moins une $\beta$-dicétone par rapport au polymère chloré.

**18.** Compositions selon l'une des revendications 9 à 17, caractérisées en ce qu'elles comprennent de 0,005% à 1% en poids de polyol par rapport au polymère chloré.

**19.** Compositions selon la revendication 18, caractérisées en ce qu'elles comprennent de 0,01% à 0,6% en poids de polyol par rapport au polymère chloré.

**20.** Compositions selon l'une des revendications 9 à 19, caractérisées en ce qu'elles comprennent de 0,05% à 5% en poids d'au moins un phosphite organique par rapport au polymère chloré.

**21.** Compositions selon la revendication 20, caractérisées en ce qu'elles comprennent de 0,1% à 2% en poids d'au moins un phosphite organique par rapport au polymère chloré.

**22.** Utilisation des composés de formule générale (VI):

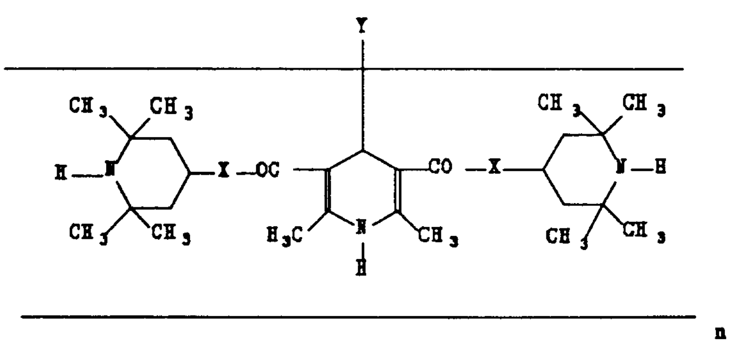

(VI)

dans laquelle:

- n est 1 ou 2,
- X représente un atome d'oxygène ou un radical N-$R_2$ dans lequel $R_2$ représente un atome d'hydrogène ou un radical alkyle ayant 1 à 18 atomes de carbone,
- Y représente un atome d'hydrogène, un radical alkyle ayant 1 à 18 atomes de carbone ou un radical phényle,
- lorsque n = 2, Y représente un radical alkylène, linéaire ou ramifié, ayant 1 à 12 atomes de carbone,

comme antioxydants dans les polymères organiques et plus particulièrement dans les polyoléfines, les polystyrènes, les polyalcadiènes, les polyuréthanes, les polyamides, les polyesters, les polycarbonates, les polysulfones, les polyéthers-sulfones, les polyéthers-cétones, les polymères acryliques, les polymères halogénés, les copolymères et les mélanges de ces polymères.

**23.** Utilisation selon la revendication 22 des composés de formule (VI), caractérisée en ce que ces composés représentent de 0,01% à 5% en poids par rapport au poids du polymère à stabiliser.

**24.** Utilisation selon la revendication 23 des composés de formule (VI), caractérisée en ce que ces composés représentent de 0,05% à 2% en poids par rapport au poids du polymère à stabiliser.

**25.** Utilisation selon l'une des revendications 22 à 24 des composés de formule (VI), caractérisée en ce que ces composés sont associés à des phosphites organiques.

**26.** Utilisation selon la revendication 25 des composés de formule (VI), caractérisée en ce que ces composés sont associés au phosphite de tris(ditertiobutyl-2,4-phényle).

**Revendications pour l'Etat contractant suivant : ES**

**1.** Procédé de préparation des composés à fonction dihydro-1,4 pyridine de formule générale (Ia) et (Ib) :

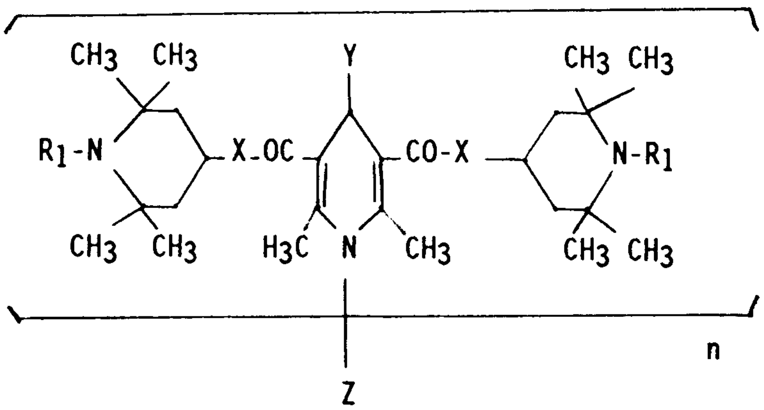

(Ia)

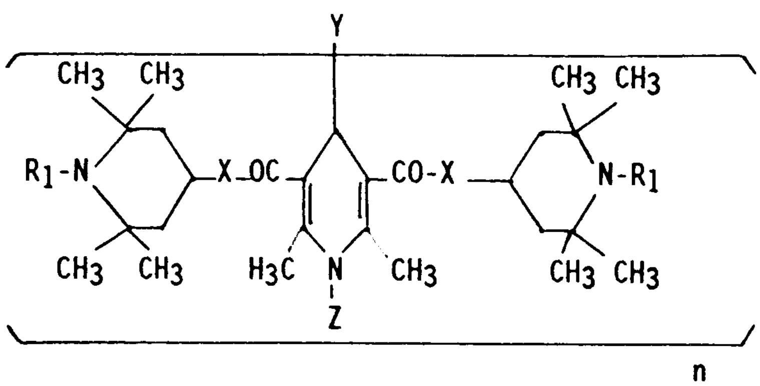

(Ib)

dans lesquelles :

- n est 1 ou 2
- $R_1$ représente un atome d'hydrogène, un radical méthyle ou un radical acétyle ;
- X représente un atome d'oxygène ou un radical N-$R_2$ dans lequel $R_2$ représente un atome d'hydrogène ou un radical alkyle ayant 1 à 18 atomes de carbone ;
- Y représente un atome d'hydrogène, un radical alkyle ayant 1 à 18 atomes de carbone ou un radical phényle ;
- Z représente un atome d'hydrogène ou un radical alkyle ayant 1 à 18 atomes de carbone ;
- lorsque n = 2 l'un des 2 symboles Y et Z peut représenter un radical alkylène ayant 1 à 12 atomes de carbone ;

caractérisé en ce que l'on fait réagir un ester acétoacétique ou un acétoacétamide de formule (II)

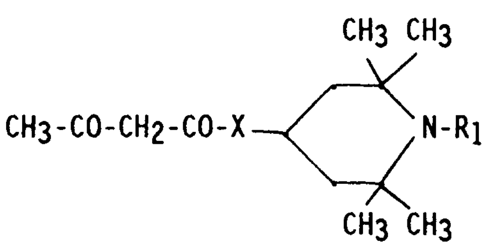

(II)

avec un aldéhyde de formule (III) $Y\text{-}(\text{-}CHO)_n$ et une amine de formule (IV) $Z\text{-}(\text{-}NH_2)_n$, les différents symboles n, X et $R_1$, Y et Z dans les formules (II), (III) et (IV) ayant les significations indiquées pour les formules (Ia) et (Ib).

**2.** Procédé selon la revendication 1, caractérisé en ce qu'il est appliqué à la préparation des composés de formule (Ia) ou (Ib) suivants :

- la diméthyl-2,6 bis[(pentaméthyl-1,2,2,6,6 pipéridinyl-4)-oxycarbonyl]-3,5 dihydro-1,4 pyridine ;
- la diméthyl-2,6 bis[(tétraméthyl-2,2,6,6 pipéridinyl-4)-oxycarbonyl]-3,5 dihydro-1,4 pyridine ;
- la diméthyl-2,6 bis[(tétraméthyl-2,2,6,6 pipéridinyl-4)-amino carbonyl]-3,5 dihydro-1,4 pyridine ;
- la diméthyl-2,6 bis[(tétraméthyl-2,2,6,6 pipéridinyl-4)-N-butylaminocarbonyl]-3,5 dihydro-1,4 pyridine ;
- la bis[diméthyl-2,6 bis[(pentaméthyl-1,2,2,6,6 pipéridinyl-4)-oxycarbonyl]-3,5 dihydro-1,4 pyridinyl-4]-1,6 hexane.

**3.** Procédé selon la revendication 1, caractérisé en ce qu'il est appliqué à la préparation des composés de formule générale (VI) :

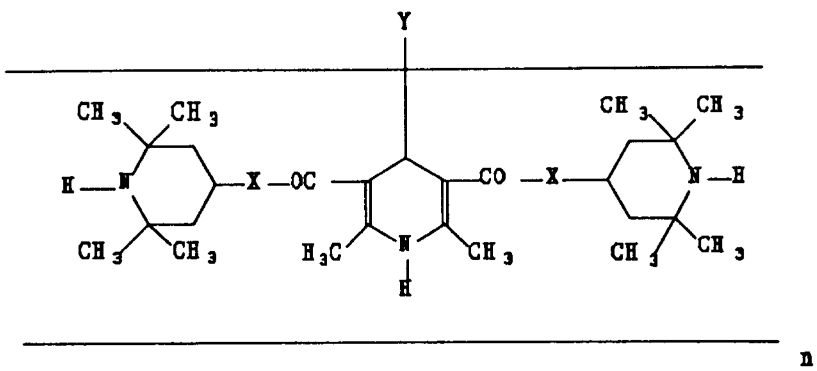

(VI)

dans laquelle:

- n est 1 ou 2,
- X représente un atome d'oxygène ou un radical $N\text{-}R_2$ dans lequel $R_2$ représente un atome d'hydrogène ou un radical alkyle ayant 1 à 18 atomes de carbone,
- Y représente un atome d'hydrogène, un radical alkyle ayant 1 à 18 atomes de carbone ou un radical phényle,
- lorsque n = 2, Y représente un radical alkylène, linéaire ou ramifié, ayant 1 à 12 atomes de carbone.

**4.** Compositions de polymère organique stabilisé contre les effets néfastes de la lumière et des rayons ultra-violets par une quantité efficace d'au moins un composé de formule (Ia) ou (Ib) préparé selon l'une des revendications 1 ou 2.

**5.** Compositions selon la revendication 4, caractérisées en ce que le polymère organique est choisi parmi les polyoléfines, les polystyrènes, les polyalcadiènes, les polyuréthanes, les polyamides, les polyesters, les polycarbonates, les polysulfones, les polyéthers-sulfones, les polyéthers-cétones, les polymères acryliques, les polymères halogénés, leurs copolymères et leurs mélanges.

**6.** Compositions selon l'une des revendications 4 et 5, caractérisées en ce qu'elles contiennent de 0,004 à 20 milliéquivalents de fonction tétraméthyl-2,2,6,6-pipéridinyle pour 100 g de polymère.

**7.** Compositions selon la revendication 6, caractérisées en ce qu'elles contiennent de 0,02 à 4 milliéquivalents de fonction tétraméthyl-2,2,6,6-pipéridinyle pour 100 g de polymère.

**8.** Utilisation des composés de formules (Ia) et (Ib) préparés selon l'une des revendications 1 et 2 comme stabilisants thermiques et stabilisants UV dans les polymères chlorés.

9. Compositions à base de polymère chloré stabilisé selon la revendication 8, caractérisées en ce qu'elles contiennent:
   a) une quantité efficace d'au moins un composé organique du zinc,
   b) une quantité efficace d'au moins un composé organique du calcium, du baryum, du magnésium ou du strontium et/ou d'une hydrotalcite,
   c) une quantité efficace d'au moins un composé à fonction dihydro-1,4-pyridine de formule (Ia) ou (Ib).

10. Compositions selon la revendication 9, caractérisées en ce qu'elles comprennent de 0,005% à 5% en poids de composé de formule (Ia) ou (Ib) par rapport au polymère chloré.

11. Compositions selon la revendication 10, caractérisées en ce qu'elles comprennent de 0,01% à 2% en poids de composé de formule (Ia) ou (Ib) par rapport au polymère chloré.

12. Compositions selon l'une des revendications 9 à 11, caractérisées en ce qu'elles comprennent:
   - de 0,005% à 1% en poids d'au moins un composé organique du zinc par rapport au polymère chloré,
   - de 0,005% à 5% en poids d'au moins un composé organique du calcium, du baryum, du magnésium et du strontium ou une hydrotalcite par rapport au polymère chloré.

13. Compositions selon la revendication 12, caractérisées en ce qu'elles comprennent:
   - de 0,005% à 1% en poids d'au moins un composé organique du zinc par rapport au polymère chloré,
   - de 0,02% à 2% en poids d'au moins un composé organique du calcium, du baryum, du magnésium et du strontium ou une hydrotalcite par rapport au polymère chloré.

14. Compositions selon la revendication 12, caractérisées en ce qu'elles comprennent:
   - de 0,01% à 0,6% en poids d'au moins un composé organique du zinc par rapport au polymère chloré,
   - de 0,005% à 5% en poids d'au moins un composé organique du calcium, du baryum, du magnésium et du strontium ou une hydrotalcite par rapport au polymère chloré.

15. Compositions selon l'une des revendications 13 et 14, caractérisées en ce qu'elles comprennent:
   - de 0,01% à 0,6% en poids d'au moins un composé organique du zinc par rapport au polymère chloré,
   - de 0,02% à 2% en poids d'au moins un composé organique du calcium, du baryum, du magnésium et du strontium ou une hydrotalcite par rapport au polymère chloré.

16. Compositions selon l'une des revendications 9 à 15, caractérisées en ce qu'elles comprennent de 0,005% à 5% en poids d'au moins une $\beta$-dicétone par rapport au polymère chloré.

17. Compositions selon la revendication 16, caractérisées en ce qu'elles comprennent de 0,01% à 2% en poids d'au moins une $\beta$-dicétone par rapport au polymère chloré.

18. Compositions selon l'une des revendications 9 à 17, caractérisées en ce qu'elles comprennent de 0,005% à 1% en poids de polyol par rapport au polymère chloré.

19. Compositions selon la revendication 18, caractérisées en ce qu'elles comprennent de 0,01% à 0,6% en poids de polyol par rapport au polymère chloré.

20. Compositions selon l'une des revendications 9 à 19, caractérisées en ce qu'elles comprennent de 0,05% à 5% en poids d'au moins un phosphite organique par rapport au polymère chloré.

21. Compositions selon la revendication 20, caractérisées en ce qu'elles comprennent de 0,1% à 2% en poids d'au moins un phosphite organique par rapport au polymère chloré.

**22.** Utilisation des composés de formule générale (VI):

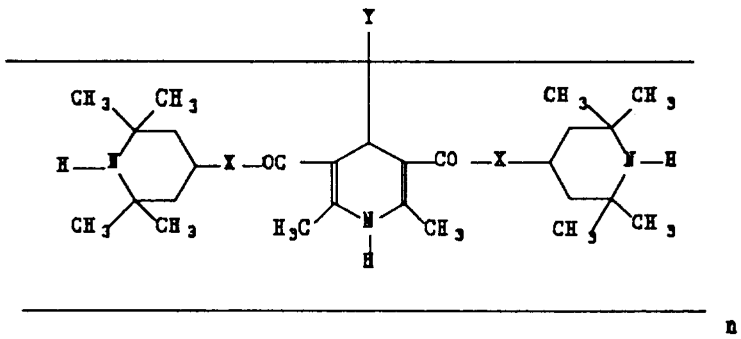

(VI)

dans laquelle:
- n est 1 ou 2,
- X représente un atome d'oxygène ou un radical N-$R_2$ dans lequel $R_2$ représente un atome d'hydrogène ou un radical alkyle ayant 1 à 18 atomes de carbone,
- Y représente un atome d'hydrogène, un radical alkyle ayant 1 à 18 atomes de carbone ou un radical phényle,
- lorsque n = 2, Y représente un radical alkylène, linéaire ou ramifié, ayant 1 à 12 atomes de carbone,

comme antioxydants dans les polymères organiques et plus particulièrement dans les polyoléfines, les polystyrènes, les polyalcadiènes, les polyuréthanes, les polyamides, les polyesters, les polycarbonates, les polysulfones, les polyéthers-sulfones, les polyéthers-cétones, les polymères acryliques, les polymères halogénés, les copolymères et les mélanges de ces polymères.

**23.** Utilisation selon la revendication 22 des composés de formule (VI), caractérisée en ce que ces composés représentent de 0,01% à 5% en poids par rapport au poids du polymère à stabiliser.

**24.** Utilisation selon la revendication 23 des composés de formule (VI), caractérisée en ce que ces composés représentent de 0,05% à 2% en poids par rapport au poids du polymère à stabiliser.

**25.** Utilisation selon l'une des revendications 22 à 24 des composés de formule (VI), caractérisée en ce que ces composés sont associés à des phosphites organiques.

**26.** Utilisation selon la revendication 25 des composés de formule (VI), caractérisée en ce que ces composés sont associés au phosphite de tris(ditertiobutyl-2,4-phényle).

## Claims

**Claims for the following Contracting States : AT, BE, CH, DE, DK, FR, GB, GR, IT, LI, LU, NL, SE**

1. A 1,4-dihydropyridine-function compound having general formulas (Ia) and (Ib) :

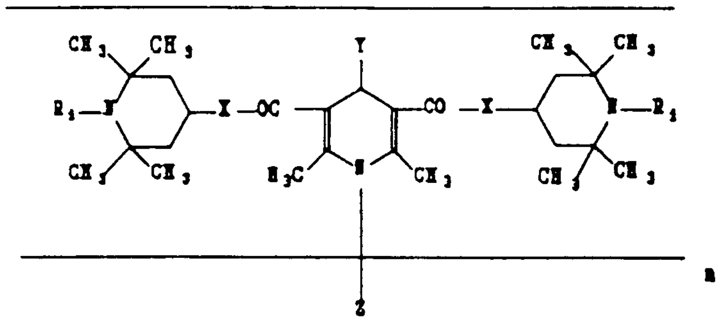

(Ia)

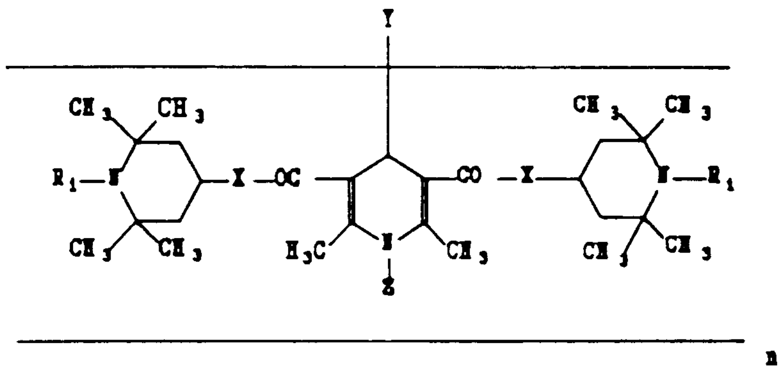

(Ib)

wherein :
- n is 1 or 2,
- $R_1$ is a hydrogen atom, a methyl radical or an acetyl radical,
- X is an oxygen atom or an N-$R_2$ radical wherein $R_2$ is a hydrogen atom or an alkyl radical having from 1 to 18 carbon atoms,
- Y is a hydrogen atom, an alkyl radical having from 1 to 18 carbon atoms or a phenyl radical,
- Z is a hydrogen atom or an alkyl radical having from 1 to 18 carbon atoms,
- when n = 2, one of the symbols Y and Z may be an alkylene radical having from 1 to 12 carbon atoms.

2. Compounds according to claim 1, selected from the group comprising :
- 2,6-dimethyl-3,5-bis[(1,2,2,6,6-pentamethyl-4-piperidyl)-oxycarbonyl]-1,4-dihydropyridine ;
- 2,6-dimethyl-3,5-bis[(2,2,6,6-tetramethyl-4-piperidyl)-oxycarbonyl]-1,4-dihydropyridine ;
- 2,6-dimethyl-3,5-bis[(2,2,6,6-tetramethyl-4-piperidyl)-aminocarbonyl]-1,4-dihydropyridine ;
- 2,6-dimethyl-3,5-bis[(2,2,6,6-tetramethyl-4-piperidyl)-N-butylaminocarbonyl]-1,4-dihydropyridine.
- 1,6-bis[2,6-dimethyl-3,5-bis[(1,2,2,6,6-pentamethyl-4-piperidyl)oxycarbonyl]-1,4-dihydro-4-pyridyl]-hexane.

3. A process for the preparation of compounds of formulas (Ia) and (Ib) according to one of claims 1 and 2, comprising reacting an acetoacetic ester or an acetoacetamide of the formula (II) :

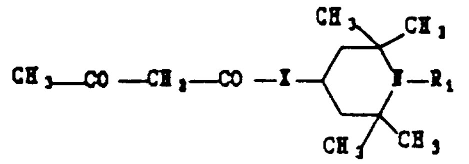

(II)

with an aldehyde of formula (III) Y-(-CHO)$_n$ and an amine of formula (IV) Z-(-$NH_2$)n, the different symbols n, X and $R_1$, Y and Z in formulas (II), (III) and (IV) having the meanings indicated in claim 1 for the formulas (Ia) and (Ib).

**4.** Organic polymer compositions stabilized against harmful effects of light and of ultraviolet rays by an effective amount of at least one compound of formula (Ia) or (Ib) according to one of claims 1 or 2.

**5.** Compositions according to claim 4, wherein the organic polymer is chosen among polyolefins, polystyrenes, polyalkadienes, polyurethanes, polyamides, polyesters, polycarbonates, polysulfones, polyethersulfones, polyetherketones, acrylic polymers, halogenated polymers, their copolymers or mixtures thereof.

**6.** Compositions according to one of claims 4 and 5, comprising from 0.004 to 20 milliequivalents of 2,2,6,6-tetramethylpiperidyl function per 100 g of polymer.

**7.** Compositions according to claim 6, comprising from 0.02 to 4 milliequivalents of 2,2,6,6-tetramethylpiperidyl function per 100 g of polymer.

**8.** Use of compounds of formulas (Ia) and (Ib) according to one of claims 1 and 2 as heat and UV stabilizers in the chlorinated polymers.

**9.** Stabilized chlorinated polymer-based compositions according to claim 8, comprising :
a) an effective amount of at least one organozinc compound,
b) an effective amount of at least one organic compound of calcium, barium, magnesium or strontium and/or of a hydrotalcite,
c) an effective amount of at least one 1,4-dihydropyridine-function compound of formula (Ia) or (Ib).

**10.** Compositions according to claim 9, comprising from 0.005 % to 5 % by weight of compound of formula (Ia) or (Ib) relative to the chlorinated polymer.

**11.** Compositions according to claim 10, comprising from 0.01 % to 2 % by weight of compound of formula (Ia) or (Ib) relative to the chlorinated polymer.

**12.** Compositions according to one of claims 9 to 11, comprising :
- from 0.005 % to 1 % by weight of at least one organozinc compound relative to the chlorinated polymer,
- from 0.005 % to 5 % by weight of at least one organic compound of calcium, barium, magnesium and strontium or one hydrotalcite relative to the chlorinated polymer.

**13.** Compositions according to claim 12, comprising :
- from 0.005 % to 1 % by weight of at least one organozinc compound relative to the chlorinated polymer,
- from 0.02 % to 2 % by weight of at least one organic compound of calcium, barium, magnesium or strontium or one hydrotalcite relative to the chlorinated polymer.

**14.** Compositions according to claim 12, comprising :
- from 0.01 % to 0.6 % by weight of at least one organozinc compound relative to the chlorinated polymer,

- from 0.005 % to 5 % by weight of at least one organic compound of calcium, barium, magnesium and strontium or one hydrotalcite relative to the chlorinated polymer.

**15.** Compositions according to one of claims 13 and 14, comprising :
- from 0.01 % to 0.6 % by weight of at least one organozinc compound relative to the chlorinated polymer,
- from 0.02 % to 2 % by weight of at least one organic compound of calcium, barium, magnesium and strontium or one hydrotalcite relative to the chlorinated polymer.

**16.** Compositions according to one of claims 9 to 15, comprising from 0.005 % to 5 % by weight of at least one $\beta$-diketone relative to the chlorinated polymer.

**17.** Compositions according to claim 16, comprising from 0.01 % to 2 % by weight of at least one $\beta$-diketone relative to the chlorinated polymer.

**18.** Compositions according to one of claims 9 to 17, comprising from 0.005 % to 1 % by weight of polyol relative to the chlorinated polymer.

**19.** Compositions according to claim 18, comprising from 0.01 % to 0.6 % by weight of polyol relative to the chlorinated polymer.

**20.** Compositions according to one of claims 9 to 19, comprising from 0.05 % to 5 % by weight of at least one organic phosphite relative to the chlorinated polymer.

**21.** Compositions according to claim 20, comprising from 0.1 % to 2 % by weight of at least one organic phosphite relative to the chlorinated polymer.

**22.** Use of compounds of general formula (VI) :

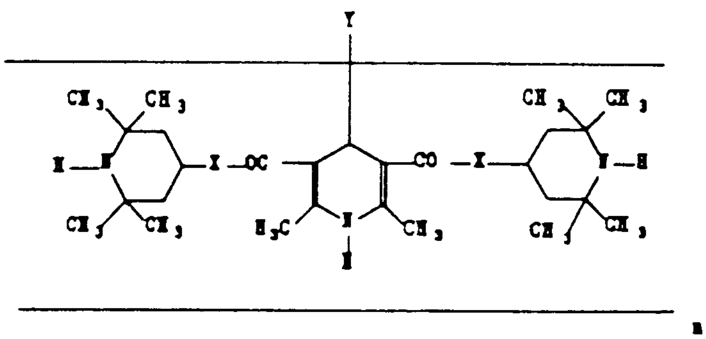

(VI)

wherein :
- n is 1 or 2,
- X is an oxygen atom or a radical N-$R_2$ in which $R_2$ is a hydrogen atom or an alkyl radical having from 1 to 18 carbon atoms,
- Y is a hydrogen atom, an alkyl radical having from 1 to 18 carbon atoms or a phenyl radical,
- when n = 2, Y is an alkylene radical, linear or branched, having from 1 to 12 carbon atoms,

as antioxidants in organic polymers and more particularly in polyolefins, polystyrenes, polyalkadienes, polyurethanes, polyamides, polyesters, polycarbonates, polysulfones, polyethersulfones, polyetherketones, acrylic polymers, halogenated polymers, copolymers and mixtures of these polymers.

**23.** Use according to claim 22 of compounds of formula (VI), wherein these compounds represent from 0.01 % to 5 % by weight relative to the weight of the polymer to be stabilized.

**24.** Use according to claim 23 of compounds of formula (VI), wherein these compounds represent from 0.05 % to 2 % by weight relative to the weight of the polymer to be stabilized.

**25.** Use according to one of claims 22 to 24 of compounds of formula (VI), wherein these compounds are associated with organic phosphites.

**26.** Use according to claim 25 of compounds of formula (VI), wherein these compounds are associated with the tris(ditertiobutyl-2,4-phenyl)phosphite.

**Claims for the following Contracting State : ES**

**1.** A process for the preparation of 1,4-dihydropyridine-function compounds having general formulas (Ia) and (Ib) :

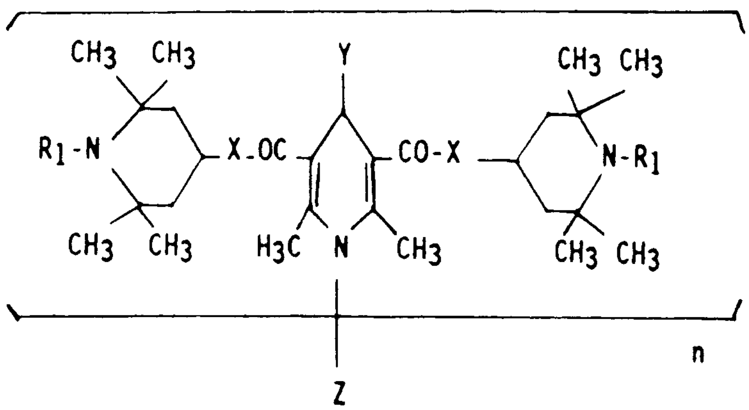

(Ia)

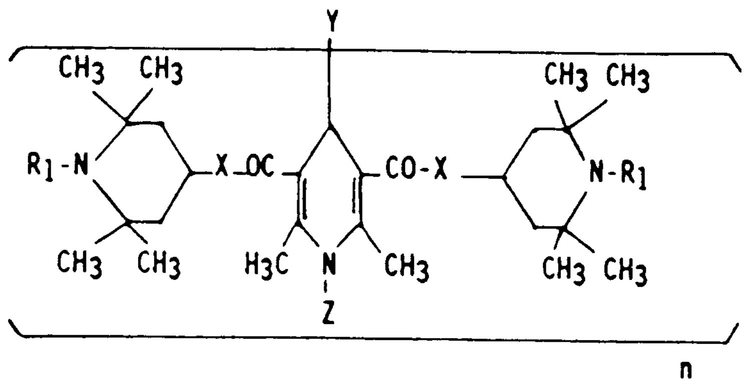

(Ib)

wherein :

- n is 1 or 2,
- $R_1$ is a hydrogen atom, a methyl radical or an acetyl radical,
- X is an oxygen atom or an N-$R_2$ radical wherein $R_2$ is a hydrogen atom or an alkyl radical having from 1 to 18 carbon atoms,
- Y is a hydrogen atom, an alkyl radical having from 1 to 18 carbon atoms or a phenyl radical,
- Z is a hydrogen atom or an alkyl radical having from 1 to 18 carbon atoms,
- when n = 2, one of the symbols Y and Z may be an alkylene radical having from 1 to 12 carbon atoms,

comprising reacting an acetoacetic ester or an acetoacetamide of the formula (II)

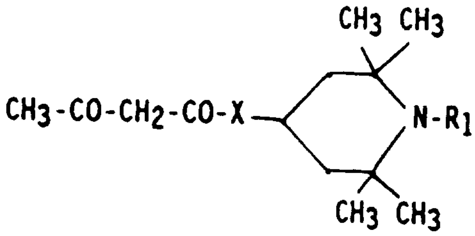

(II)

with an aldehyde of formula (III) Y-(-CHO)n and an amine of formula (IV) Z-(-$NH_2$)n, the different symbols n, X and $R_1$, Y and Z in formulas (II), (III) and (IV) having the meanings indicated for the formulas (Ia) and (Ib).

2. A process according to claim 1, applied to the preparation of compounds of formula (Ia) or (Ib) as follows :
   - 2,6-dimethyl-3,5-bis[(1,2,2,6,6-pentamethyl-4-piperidyl)-oxycarbonyl]-1,4-dihydropyridine ;
   - 2,6-dimethyl-3,5-bis[(2,2,6,6-tetramethyl-4-piperidyl)-oxycarbonyl]-1,4-dihydropyridine ;
   - 2,6-dimethyl-3,5-bis[(2,2,6,6-tetramethyl-4-piperidyl)-aminocarbonyl]-1,4-dihydropyridine ;
   - 2,6-dimethyl-3,5-bis[(2,2,6,6-tetramethyl-4-piperidyl)-N-butylaminocarbonyl]-1,4-dihydropyridine.
   - 1,6-bis[2,6-dimethyl-3,5-bis[(1,2,2,6,6-pentamethyl-4-piperidyl)oxycarbonyl]-1,4-dihydro-4-pyridyl]-hexane.

3. A process according to claim 1, applied to the preparation of compounds of the general formula (VI) :

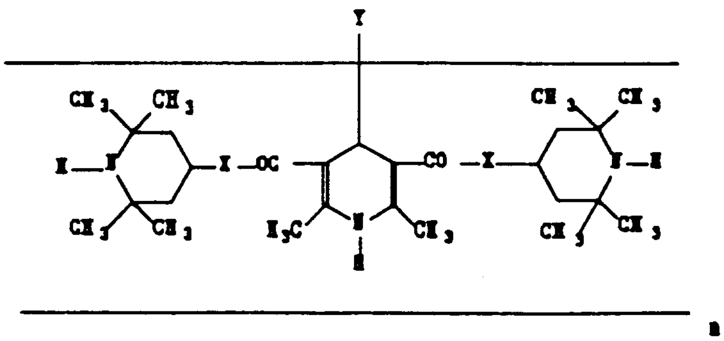

(VI)

wherein :
   - n is 1 or 2,
   - X is an oxygen atom or a radical N-$R_2$ in which $R_2$ is a hydrogen atom or an alkyl radical having from 1 to 18 carbon atoms,
   - Y is a hydrogen atom, an alkyl radical having from 1 to 18 carbon atoms or a phenyl radical,
   - when n = 2, Y is an alkylene radical, linear or branched, having from 1 to 12 carbon atoms.

4. Organic polymer compositions stabilized against harmful effects of light and of ultraviolet rays by an effective amount of at least one compound of formula (Ia) or (Ib) prepared according to one of claims 1 or 2.

5. Compositions according to claim 4, wherein the organic polymer is chosen among polyolefins, polystyrenes, polyalkadienes, polyurethanes, polyamides, polyesters, polycarbonates, polysulfones, polyethersulfones, polyetherketones, acrylic polymers, halogenated polymers, their copolymers or mixtures thereof.

6. Compositions according to one of claims 4 and 5, comprising from 0.004 to 20 milliequivalents of 2,2,6,6-tetramethylpiperidyl function per 100 g of polymer.

7. Compositions according to claim 6, comprising from 0.02 to 4 milliequivalents of 2,2,6,6-tetramethylpiperidyl function per 100 g of polymer.

**8.** Use of compounds of formulas (Ia) and (Ib) according to one of claims 1 and 2 as heat and UV stabilizers in the chlorinated polymers.

**9.** Stabilized chlorinated polymer-based compositions according to claim 8, comprising :
a) an effective amount of at least one organozinc compound,
b) an effective amount of at least one organic compound of calcium, barium, magnesium or strontium and/or of a hydrotalcite,
c) an effective amount of at least one 1,4-dihydropyridine-function compound of formula (Ia) or (Ib).

**10.** Compositions according to claim 9, comprising from 0.005 % to 5 % by weight of compound of formula (Ia) or (Ib) relative to the chlorinated polymer.

**11.** Compositions according to claim 10, comprising from 0.01 % to 2 % by weight of compound of formula (Ia) or (Ib) relative to the chlorinated polymer.

**12.** Compositions according to one of claims 9 to 11, comprising :
- from 0.005 % to 1 % by weight of at least one organozinc compound relative to the chlorinated polymer,
- from 0.005 % to 5 % by weight of at least one organic compound of calcium, barium, magnesium and strontium or one hydrotalcite relative to the chlorinated polymer.

**13.** Compositions according to claim 12, comprising :
- from 0.005 % to 1 % by weight of at least one organozinc compound relative to the chlorinated polymer,
- from 0.02 % to 2 % by weight of at least one organic compound of calcium, barium, magnesium or strontium or one hydrotalcite relative to the chlorinated polymer.

**14.** Compositions according to claim 12, comprising :
- from 0.01 % to 0.6 % by weight of at least one organozinc compound relative to the chlorinated polymer,
- from 0.005 % to 5 % by weight of at least one organic compound of calcium, barium, magnesium and strontium or one hydrotalcite relative to the chlorinated polymer.

**15.** Compositions according to one of claims 13 and 14, comprising :
- from 0.01 % to 0.6 % by weight of at least one organozinc compound relative to the chlorinated polymer,
- from 0.02 % to 2 % by weight of at least one organic compound of calcium, barium, magnesium and strontium or one hydrotalcite relative to the chlorinated polymer.

**16.** Compositions according to one of claims 9 to 15, comprising from 0.005 % to 5 % by weight of at least one $\beta$-diketone relative to the chlorinated polymer.

**17.** Compositions according to claim 16, comprising from 0.01 % to 2 % by weight of at least one $\beta$-diketone relative to the chlorinated polymer.

**18.** Compositions according to one of claims 9 to 17, comprising from 0.005 % to 1 % by weight of polyol relative to the chlorinated polymer.

**19.** Compositions according to claim 18, comprising from 0.01 % to 0.6 % by weight of polyol relative to the chlorinated polymer.

**20.** Compositions according to one of claims 9 to 19, comprising from 0.05 % to 5 % by weight of at least one organic phosphite relative to the chlorinated polymer.

**21.** Compositions according to claim 20, comprising from 0.1 % to 2 % by weight of at least one organic phosphite relative to the chlorinated polymer.

**22.** Use of compounds of general formula (VI) :

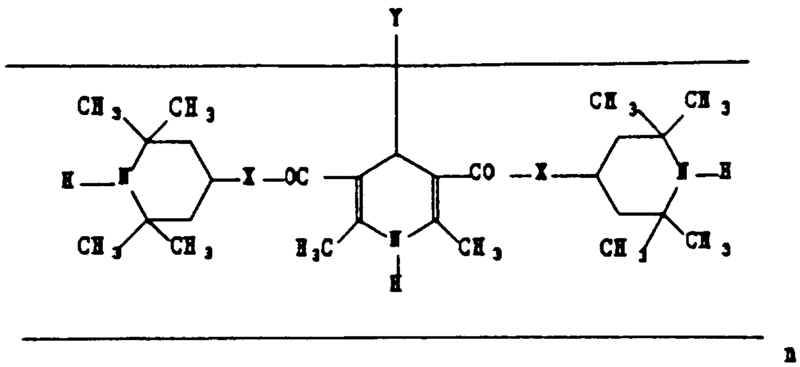

(VI)

wherein :

- n is 1 or 2,
- X is an oxygen atom or a radical N-$R_2$ in which $R_2$ is a hydrogen atom or an alkyl radical having from 1 to 18 carbon atoms,
- Y is a hydrogen atom, an alkyl radical having from 1 to 18 carbon atoms or a phenyl radical,
- when n = 2, Y is an alkylene radical, linear or branched, having from 1 to 12 carbon atoms,

as antioxidants in organic polymers and more particularly in polyolefins, polystyrenes, polyalkadienes, polyurethanes, polyamides, polyesters, polycarbonates, polysulfones, polyethersulfones, polyetherketones, acrylic polymers, halogenated polymers, copolymers and mixtures of these polymers.

**23.** Use according to claim 22 of compounds of formula (VI), wherein these compounds represent from 0.01 % to 5 % by weight relative to the weight of the polymer to be stabilized.

**24.** Use according to claim 23 of compounds of formula (VI), wherein these compounds represent from 0.05 % to 2 % by weight relative to the weight of the polymer to be stabilized.

**25.** Use according to one of claims 22 to 24 of compounds of formula (VI), wherein these compounds are associated with organic phosphites.

**26.** Use according to claim 25 of compounds of formula (VI), wherein these compounds are associated with the tris(ditertiobutyl-2,4-phenyl)phosphite.

**Patentansprüche**

**Patentansprüche für folgende Vertragsstaaten : AT, BE, CH, DE, DK, FR, GB, GR, IT, LI, LU, NL, SE**

**1.** Verbindungen mit 1,4-Dihydropyridin-Funktion der allgemeinen Formeln (Ia) und Ib)

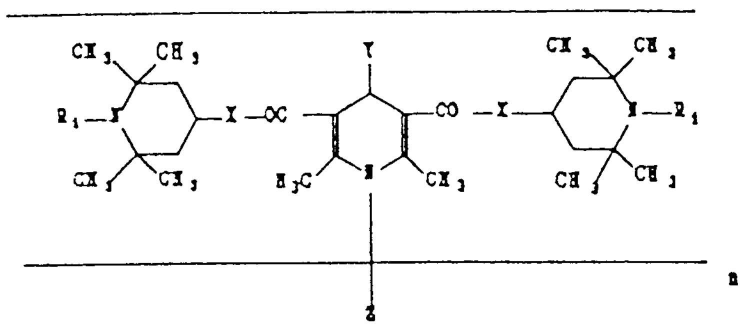

(Ia)

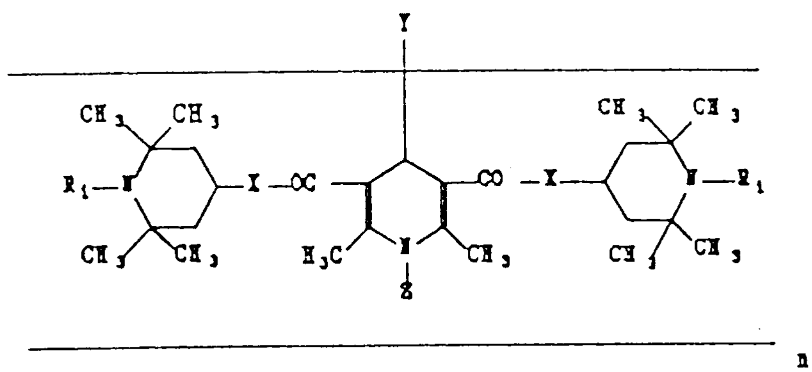

(Ib)

in denen

- n 1 oder 2 ist,
- $R_1$ ein Wasserstoffatom, einen Methylrest oder einen Acetylrest darstellt,
- X ein Sauerstoffatom oder einen Rest N-$R_2$ bedeutet, worin $R_2$ ein Wasserstoffatom oder ein Alkylrest mit 1 bis 18 Kohlenstoffatomen ist,
- Y ein Wasserstoffatom, einen Alkylrest mit 1 bis 18 Kohlenstoffatomen oder einen Phenylrest darstellt,
- Z ein Wasserstoffatom oder ein Alkylrest mit 1 bis 18 Kohlenstoffatomen ist,
- wenn n = 2 ist, eines der zwei Symbole Y und Z einen Alkylenrest mit 1 bis 12 Kohlenstoffatomen darstellen kann.

**2.** Verbindungen nach Anspruch 1, dadurch gekennzeichnet, daß sie aus der Gruppe ausgewählt werden, die umfaßt:

- 2,6-Dimethyl-3,5-bis-[(1,2,2,6,6-pentamethyl-4-piperidinyl)-oxycarbonyl]-1,4-dihydropyridin;
- 2,6-Dimethyl-3,5-bis-[(2,2,6,6-tetramethyl-4-piperidinyl)-oxycarbonyl]-1,4-dihydropyridin;
- 2,6-Dimethyl-3,5-bis-[(2,2,6,6-tetramethyl-4-piperidinyl)-aminocarbonyl]-1,4-dihydropyridin;
- 2,6-Dimethyl-3,5-bis-[(2,2,6,6-tetramethyl-4-piperidinyl)-N-butylaminocarbonyl]-1,4-dihydropyridin;
- 1,6-Bis-{2,6-Dimethyl-3,5-bis-[(1,2,2,6,6-pentamethyl-4-piperidinyl)-oxycarbonyl]-1,4-dihydro-4-pyridinyl}-hexan.

**3.** Verfahren zur Herstellung der Verbindungen der Formeln (Ia) und (Ib) nach einem der Ansprüche 1 und 2, dadurch gekennzeichnet, daß man einen Acetessigsäureester oder ein Acetessigsäureamid der Formel (II)

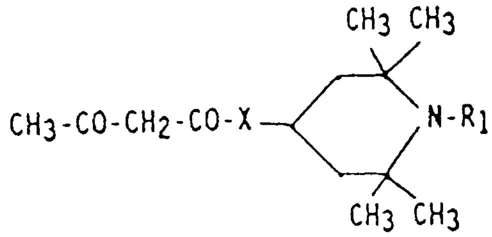

(II)

mit einem Aldehyd der Formel (III) Y-(-CHO)$_n$ und einem Amin der Formel (IV) Z-(-NH$_2$)$_n$ zur Reaktion bringt, wobei die unterschiedlichen Symbole n, X und $R_1$, Y und Z in den Formeln (II), (III) und (IV) die vorstehend in Anspruch (I) bei den Formeln (Ia) und (Ib) angegebenen Bedeutungen besitzen.

**4.** Organische Polymer-Zusammensetzungen, stabilisiert gegen schädliche Einwirkungen von Licht und ultravioletter Strahlung durch eine wirksame Menge von mindestens einer Verbindung der Formeln (Ia) oder (Ib) nach einem der Ansprüche 1 oder 2.

**5.** Zusammensetzungen nach Anspruch 4, dadurch gekennzeichnet, daß das organische Polymer unter den Polyolefinen, den Polystyrolen, den Polyalkadienen, den Polyurethanen, den Polyamiden, den Polyestern, den Polycarbonaten, den Polysulfonen, den Polyether-sulfonen, den Polyether-ketonen, den Acrylpolymeren, den halogenierten Polymeren, ihren Copolymeren und ihren Mischungen ausgewählt wird.

**6.** Zusammensetzungen nach einem der Ansprüche 4 und 5, dadurch gekennzeichnet, daß sie 0,004 bis 20 Milliäquivalente der Funktion 2,2,6,6-Tetramethyl-piperidinyl pro 100 g Polymer enthalten.

**7.** Zusammensetzungen nach Anspruch 6, dadurch gekennzeichnet, daß sie 0,02 bis 4 Milliäquivalente der Funktion 2,2,6,6-Tetramethyl-piperidinyl pro 100 g Polymer enthalten.

**8.** Verwendung der Verbindungen der Formeln (Ia) und (Ib) nach einem der Ansprüche 1 und 2 als thermische Stabilisatoren und als UV-Stabilisatoren in chlorierten Polymeren.

**9.** Zusammensetzungen auf der Basis von stabilisiertem, chloriertem Polymer nach Anspruch 8, dadurch gekennzeichnet, daß sie enthalten:

a) eine wirksame Menge von mindestens einer organischen Verbindung des Zinks,

b) eine wirksame Menge von mindestens einer organischen Verbindung des Calciums, Bariums, Magnesiums oder Strontiums und/oder einem Hydrotalcit,

c) eine wirksame Menge von mindestens einer Verbindung mit 1,4-Dihydropyridin-Funktion der Formeln (Ia) oder (Ib).

**10.** Zusammensetzungen nach Anspruch 9, dadurch gekennzeichnet, daß sie 0,005 Gew.-% bis 5 Gew.-% der Verbindung der Formeln (Ia) oder (Ib) im Verhältnis zum chlorierten Polymer umfassen.

**11.** Zusammensetzungen nach Anspruch 10, dadurch gekennzeichnet, daß sie 0,01 Gew.-% bis 2 Gew.-% der Verbindung der Formeln (Ia) oder (Ib) im Verhältnis zum chlorierten Polymer umfassen.

**12.** Zusammensetzungen nach einem der Ansprüche 9 bis 11, dadurch gekennzeichnet, daß sie umfassen:

- 0,005 Gew.-% bis 1 Gew.-% von mindestens einer organischen Verbindung des Zinks, im Verhältnis zum chlorierten Polymer,
- 0,005 Gew.-% bis 5 Gew.-% von mindestens einer organischen Verbindung des Calciums, Bariums, Magnesiums und Strontiums oder einem Hydrotalcit, im Verhältnis zum chlorierten Polymer.

**13.** Zusammensetzungen nach Anspruch 12, dadurch gekennzeichnet, daß sie umfassen:

- 0,005 Gew.-% bis 1 Gew.-% von mindestens einer organischen Verbindung des Zinks, im Verhältnis zum chlorierten Polymer,

- 0,02 Gew.-% bis 2 Gew.-% von mindestens einer organischen Verbindung des Calciums, Bariums, Magnesiums und Strontiums oder einem Hydrotalcit, im Verhältnis zum chlorierten Polymer.

**14.** Zusammensetzungen nach Anspruch 12, dadurch gekennzeichnet, daß sie umfassen:
- 0,01 Gew.-% bis 0,6 Gew.-% von mindestens einer organischen Verbindung des Zinks, im Verhältnis zum chlorierten Polymer,
- 0,005 Gew.-% bis 5 Gew.-% von mindestens einer organischen Verbindung des Calciums, Bariums, Magnesiums und Strontiums oder einem Hydrotalcit, im Verhältnis zum chlorierten Polymer.

**15.** Zusammensetzungen nach einem der Ansprüche 13 und 14, dadurch gekennzeichnet, daß sie umfassen:
- 0,01 Gew.-% bis 0,6 Gew.-% von mindestens einer organischen Verbindung des Zinks, im Verhältnis zum chlorierten Polymer,
- 0,02 Gew.-% bis 2 Gew.-% von mindestens einer organischen Verbindung des Calciums, Bariums, Magnesiums und Strontiums oder einem Hydrotalcit, im Verhältnis zum chlorierten Polymer.

**16.** Zusammensetzungen nach einem der Ansprüche 9 bis 15, dadurch gekennzeichnet, daß sie 0,005 Gew.-% bis 5 Gew.-% von mindestens einem $\beta$-Diketon im Verhältnis zum chlorierten Polymer umfassen.

**17.** Zusammensetzungen nach Anspruch 16, dadurch gekennzeichnet, daß sie 0,01 Gew.-% bis 2 Gew.-% von mindestens einem $\beta$-Diketon im Verhältnis zum chlorierten Polymer umfassen.

**18.** Zusammensetzungen nach einem der Ansprüche 9 bis 17, dadurch gekennzeichnet, daß sie 0,005 Gew.-% bis 1 Gew.-% Polyol im Verhältnis zum chlorierten Polymer umfassen.

**19.** Zusammensetzungen nach Anspruch 18, dadurch gekennzeichnet, daß sie 0,01 Gew.-% bis 0,6 Gew.-% Polyol im Verhältnis zum chlorierten Polymer umfassen.

**20.** Zusammensetzungen nach einem der Ansprüche 9 bis 19, dadurch gekennzeichnet, daß sie 0,05 Gew.-% bis 5 Gew.-% von mindestens einem organischen Phosphit im Verhältnis zum chlorierten Polymer umfassen.

**21.** Zusammensetzungen nach Anspruch 20, dadurch gekennzeichnet, daß sie 0,1 Gew.-% bis 2 Gew.-% von mindestens einem organischen Phosphit im Verhältnis zum chlorierten Polymer umfassen.

**22.** Verwendung der Verbindungen der allgemeinen Formel (VI)

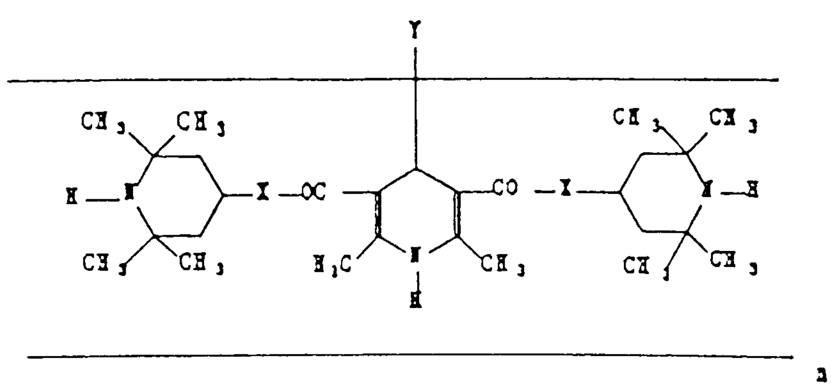

(VI)

in der
- n 1 oder 2 ist,

- X ein Sauerstoffatom oder einen Rest N-$R_2$ bedeutet, worin $R_2$ ein Wasserstoffatom oder ein Alkylrest mit 1 bis 18 Kohlenstoffatomen ist,
- Y ein Wasserstoffatom, einen Alkylrest mit 1 bis 18 Kohlenstoffatomen oder einen Phenylrest darstellt,
- wenn n = 2 ist, Y einen linearen oder verzweigten Alkylenrest mit 1 bis 12 Kohlenstoffatomen bedeutet,

als Antioxydans in organischen Polymeren und insbesondere in den Polyolefinen, den Polystyrolen, den Polyalkadienen, den Polyurethanen, den Polyamiden, den Polyestern, den Polycarbonaten, den Polysulfonen, den Polyether-sulfonen, den Polyether-ketonen, den Acrylpolymeren, den halogenierten Polymeren, den Copolymeren und den Mischungen dieser Polymeren.

**23.** Verwendung nach Anspruch 22 der Verbindungen der Formel (VI), dadurch gekennzeichnet, daß diese Verbindungen 0,01 Gew.-% bis 5 Gew.-% im Verhältnis zu dem Gewicht des zu stabilisierenden Polymer darstellen.

**24.** Verwendung nach Anspruch 23 der Verbindungen der Formel (VI), dadurch gekennzeichnet, daß diese Verbindungen 0,05 Gew.-% bis 2 Gew.-% im Verhältnis zu dem Gewicht des zu stabilisierenden Polymer darstellen.

**25.** Verwendung nach einem der Ansprüche 22 bis 24 der Verbindungen der Formel (VI), dadurch gekennzeichnet, daß diese Verbindungen mit organischen Phosphiten assoziiert sind.

**26.** Verwendung nach Anspruch 25 der Verbindungen der Formel (VI), dadurch gekennzeichnet, daß diese Verbindungen mit Tris-(2,4-Di-tert.-butylphenyl)-phosphit assoziiert sind.

**Patentansprüche für folgenden Vertragsstaat : ES**

**1.** Verfahren zur Herstellung von Verbindungen mit 1,4-Dihydropyridin-Funktion der allgemeinen Formeln (Ia) und Ib)

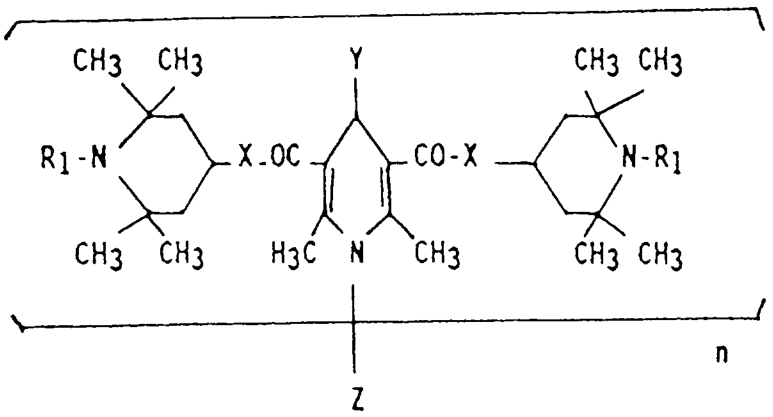

(Ia)

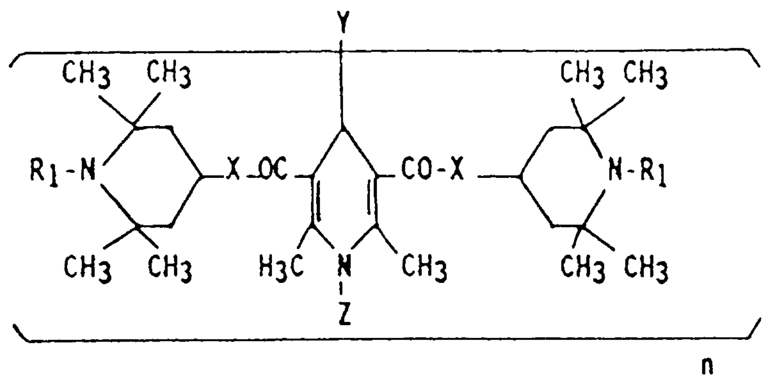

(Ib)

in denen

- n 1 oder 2 ist,
- $R_1$ ein Wasserstoffatom, einen Methylrest oder einen Acetylrest darstellt,
- X ein Sauerstoffatom oder einen Rest N-$R_2$ bedeutet, worin $R_2$ ein Wasserstoffatom oder ein Alkylrest mit 1 bis 18 Kohlenstoffatomen ist,
- Y ein Wasserstoffatom, einen Alkylrest mit 1 bis 18 Kohlenstoffatomen oder einen Phenylrest darstellt,
- Z ein Wasserstoffatom oder ein Alkylrest mit 1 bis 18 Kohlenstoffatomen ist,
- wenn n = 2 ist, eines der zwei Symbole Y und Z einen Alkylenrest mit 1 bis 12 Kohlenstoffatomen darstellen kann;

dadurch gekennzeichnet, daß man einen Acetessigsäureester oder ein Acetessigsäureamid der Formel (II)

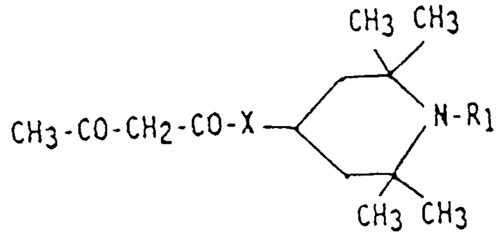

(II)

mit einem Aldehyd der Formel (III) Y-(-CHO)$_n$ und einem Amin der Formel (IV) Z-(-$NH_2$)$_n$ zur Reaktion bringt, wobei die unterschiedlichen Symbole n, X und $R_1$, Y und Z in den Formeln (II), (III) und (IV) die vorstehend bei den Formeln (Ia) und (Ib) angegebenen Bedeutungen besitzen.

2. Verfahren nach Anspruch 1, dadurch gekennzeichnet, daß es zur Herstellung der folgenden Verbindungen der Formeln (Ia) oder (Ib) eingesetzt wird:
   - 2,6-Dimethyl-3,5-bis-[(1,2,2,6,6-pentamethyl-4-piperidinyl)-oxycarbonyl]-1,4-dihydropyridin;
   - 2,6-Dimethyl-3,5-bis-[(2,2,6,6-tetramethyl-4-piperidinyl)-oxycarbonyl]-1,4-dihydropyridin;
   - 2,6-Dimethyl-3,5-bis-[(2,2,6,6-tetramethyl-4-piperidinyl)-aminocarbonyl]-1,4-dihydropyridin;
   - 2,6-Dimethyl-3,5-bis-[(2,2,6,6-tetramethyl-4-piperidinyl)-N-butylaminocarbonyl]-1,4-dihydropyridin;
   - 1,6-Bis-{2,6-Dimethyl-3,5-bis-[(1,2,2,6,6-pentamethyl-4-piperidinyl)-oxycarbonyl]-1,4-dihydro-4-pyridinyl}-hexan.

3. Verfahren nach Anspruch 1, dadurch gekennzeichnet, daß es zur Herstellung der Verbindungen der allgemeinen Formel (VI)

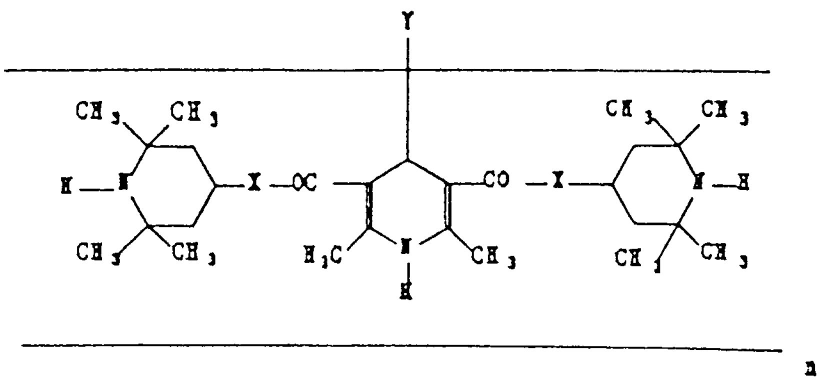

(VI)

eingesetzt wird, in der
   - n 1 oder 2 ist,
   - X ein Sauerstoffatom oder einen Rest N-$R_2$ bedeutet, worin $R_2$ ein Wasserstoffatom oder ein Alkylrest mit 1 bis 18 Kohlenstoffatomen ist,
   - Y ein Wasserstoffatom, einen Alkylrest mit 1 bis 18 Kohlenstoffatomen oder einen Phenylrest darstellt,
   - wenn n = 2 ist, Y einen linearen oder verzweigten Alkylenrest mit 1 bis 12 Kohlenstoffatomen bedeutet.

4. Organische Polymer-Zusammensetzungen, stabilisiert gegen schädliche Einwirkungen von Licht und ultravioletter Strahlung durch eine wirksame Menge von mindestens einer Verbindung der Formeln (Ia) oder (Ib) nach einem der Ansprüche 1 oder 2.

5. Zusammensetzungen nach Anspruch 4, dadurch gekennzeichnet, daß das organische Polymer unter den Polyolefinen, den Polystyrolen, den Polyalkadienen, den Polyurethanen, den Polyamiden, den Polyestern, den Polycarbonaten, den Polysulfonen, den Polyether-sulfonen, den Polyether-ketonen, den Acrylpolymeren, den halogenierten Polymeren, ihren Copolymeren und ihren Mischungen ausgewählt wird.

6. Zusammensetzungen nach einem der Ansprüche 4 und 5, dadurch gekennzeichnet, daß sie 0,004 bis 20 Milliäquivalente der Funktion 2,2,6,6-Tetramethyl-piperidinyl pro 100 g Polymer enthalten.

7. Zusammensetzungen nach Anspruch 6, dadurch gekennzeichnet, daß sie 0,02 bis 4 Milliäquivalente der Funktion 2,2,6,6-Tetramethyl-piperidinyl pro 100 g Polymer enthalten.

8. Verwendung der Verbindungen der Formeln (Ia) und (Ib),hergestellt nach einem der Ansprüche 1 und 2 als thermische Stabilisatoren und als UV-Stabilisatoren in chlorierten Polymeren.

9. Zusammensetzungen auf der Basis von stabilisiertem, chloriertem Polymer nach Anspruch 8, dadurch gekennzeichnet, daß sie enthalten:
   a) eine wirksame Menge von mindestens einer organischen Verbindung des Zinks,

b) eine wirksame Menge von mindestens einer organischen Verbindung des Calciums, Bariums, Magnesiums oder Strontiums und/oder einem Hydrotalcit,
c) eine wirksame Menge von mindestens einer Verbindung mit 1,4-Dihydropyridin-Funktion der Formeln (Ia) oder (Ib).

**10.** Zusammensetzungen nach Anspruch 9, dadurch gekennzeichnet, daß sie 0,005 Gew.-% bis 5 Gew.-% der Verbindung der Formeln (Ia) oder (Ib) im Verhältnis zum chlorierten Polymer umfassen.

**11.** Zusammensetzungen nach Anspruch 10, dadurch gekennzeichnet, daß sie 0,01 Gew.-% bis 2 Gew.-% der Verbindung der Formeln (Ia) oder (Ib) im Verhältnis zum chlorierten Polymer umfassen.

**12.** Zusammensetzungen nach einem der Ansprüche 9 bis 11, dadurch gekennzeichnet, daß sie umfassen:
- 0,005 Gew.-% bis 1 Gew.-% von mindestens einer organischen Verbindung des Zinks, im Verhältnis zum chlorierten Polymer,
- 0,005 Gew.-% bis 5 Gew.-% von mindestens einer organischen Verbindung des Calciums, Bariums, Magnesiums und Strontiums oder einem Hydrotalcit, im Verhältnis zum chlorierten Polymer.

**13.** Zusammensetzungen nach Anspruch 12, dadurch gekennzeichnet, daß sie umfassen:
- 0,005 Gew.-% bis 1 Gew.-% von mindestens einer organischen Verbindung des Zinks, im Verhältnis zum chlorierten Polymer,
- 0,02 Gew.-% bis 2 Gew.-% von mindestens einer organischen Verbindung des Calciums, Bariums, Magnesiums und Strontiums oder einem Hydrotalcit, im Verhältnis zum chlorierten Polymer.

**14.** Zusammensetzungen nach Anspruch 12, dadurch gekennzeichnet, daß sie umfassen:
- 0,01 Gew.-% bis 0,6 Gew.-% von mindestens einer organischen Verbindung des Zinks, im Verhältnis zum chlorierten Polymer,
- 0,005 Gew.-% bis 5 Gew.-% von mindestens einer organischen Verbindung des Calciums, Bariums, Magnesiums und Strontiums oder einem Hydrotalcit, im Verhältnis zum chlorierten Polymer.

**15.** Zusammensetzungen nach einem der Ansprüche 13 und 14, dadurch gekennzeichnet, daß sie umfassen:
- 0,01 Gew.-% bis 0,6 Gew.-% von mindestens einer organischen Verbindung des Zinks, im Verhältnis zum chlorierten Polymer,
- 0,02 Gew.-% bis 2 Gew.-% von mindestens einer organischen Verbindung des Calciums, Bariums, Magnesiums und Strontiums oder einem Hydrotalcit, im Verhältnis zum chlorierten Polymer.

**16.** Zusammensetzungen nach einem der Ansprüche 9 bis 15, dadurch gekennzeichnet, daß sie 0,005 Gew.-% bis 5 Gew.-% von mindestens einem $\beta$-Diketon im Verhältnis zum chlorierten Polymer umfassen.

**17.** Zusammensetzungen nach Anspruch 16, dadurch gekennzeichnet, daß sie 0,01 Gew.-% bis 2 Gew.-% von mindestens einem $\beta$-Diketon im Verhältnis zum chlorierten Polymer umfassen.

**18.** Zusammensetzungen nach einem der Ansprüche 9 bis 17, dadurch gekennzeichnet, daß sie 0,005 Gew.-% bis 1 Gew.-% Polyol im Verhältnis zum chlorierten Polymer umfassen.

**19.** Zusammensetzungen nach Anspruch 18, dadurch gekennzeichnet, daß sie 0,01 Gew.-% bis 0,6 Gew.-% Polyol im Verhältnis zum chlorierten Polymer umfassen.

**20.** Zusammensetzungen nach einem der Ansprüche 9 bis 19, dadurch gekennzeichnet, daß sie 0,05 Gew.-% bis 5 Gew.-% von mindestens einem organischen Phosphit im Verhältnis zum chlorierten Polymer umfassen.

**21.** Zusammensetzungen nach Anspruch 20, dadurch gekennzeichnet, daß sie 0,1 Gew.-% bis 2 Gew.-% von mindestens einem organischen Phosphit im Verhältnis zum chlorierten Polymer umfassen.

**22.** Verwendung der Verbindungen der allgemeinen Formel (VI)

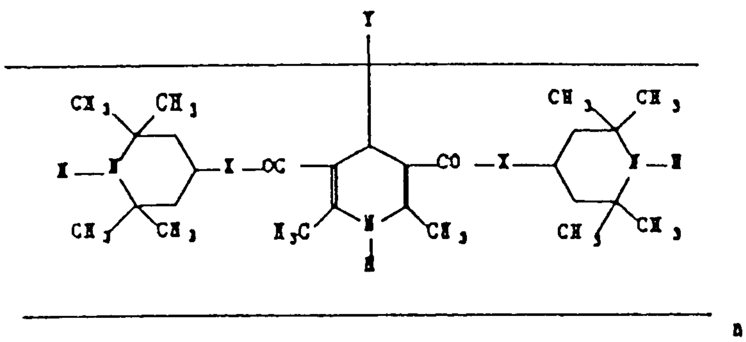

(VI)

in der

- n 1 oder 2 ist,
- X ein Sauerstoffatom oder einen Rest N-$R_2$ bedeutet, worin $R_2$ ein Wasserstoffatom oder ein Alkylrest mit 1 bis 18 Kohlenstoffatomen ist,
- Y ein Wasserstoffatom, einen Alkylrest mit 1 bis 18 Kohlenstoffatomen oder einen Phenylrest darstellt,
- wenn n = 2 ist, Y einen linearen oder verzweigten Alkylenrest mit 1 bis 12 Kohlenstoffatomen bedeutet,

als Antioxydans in organischen Polymeren und insbesondere in den Polyolefinen, den Polystyrolen, den Polyalkadienen, den Polyurethanen, den Polyamiden, den Polyestern, den Polycarbonaten, den Polysulfonen, den Polyether-sulfonen, den Polyether-ketonen, den Acrylpolymeren, den halogenierten Polymeren, den Copolymeren und den Mischungen dieser Polymeren.

**23.** Verwendung nach Anspruch 22 der Verbindungen der Formel (VI), dadurch gekennzeichnet, daß diese Verbindungen 0,01 Gew.-% bis 5 Gew.-% im Verhältnis zu dem Gewicht des zu stabilisierenden Polymer darstellen.

**24.** Verwendung nach Anspruch 23 der Verbindungen der Formel (VI), dadurch gekennzeichnet, daß diese Verbindungen 0,05 Gew.-% bis 2 Gew.-% im Verhältnis zu dem Gewicht des zu stabilisierenden Polymer darstellen.

**25.** Verwendung nach einem der Ansprüche 22 bis 24 der Verbindungen der Formel (VI), dadurch gekennzeichnet, daß diese Verbindungen mit organischen Phosphiten assoziiert sind.

**26.** Verwendung nach Anspruch 25 der Verbindungen der Formel (VI), dadurch gekennzeichnet, daß diese Verbindungen mit Tris-(2,4-Di-tert.-butylphenyl)-phosphit assoziiert sind.